# EUROPEAN PATENT SPECIFICATION

(11) **EP 1 844 074 B1**
(45) Date of publication and mention of the grant of the patent: **24.04.2013**
(21) Application number: 06709606.5
(22) Date of filing: 03.02.2006
(51) Int. Cl.: C07K 16/00, C07K 19/00, C07K 16/32, C07K 16/28, C07K 16/42, C07K 16/24, C07K 16/46, C07K 14/18, C07K 14/415, C07K 14/815

(54) **HUMAN ANTIBODIES AND PROTEINS**
MENSCHLICHE ANTIKÖRPER UND PROTEINE
PROTEINES ET ANTICORPS HUMAINS

(30) Priority: 03.02.2005 GB 0502201; 16.02.2005 GB 0503190; 05.04.2005 GB 0506945
(43) Date of publication of application: 17.10.2007
(60) Divisional application: 11177723.1
(73) Proprietor: Antitope Limited, Cambridge (GB)
(72) Inventor: JONES, Timothy David, Cambridge, CB2 4AJ (GB); BAKER, Matthew Paul, Antitope Limited, Cambridge (GB)
(74) Representative: Chapman, Paul William
(86) International application number: PCT/GB2006/000355
(87) International publication number: WO 2006/082406

(56) References cited:
- WO-A2-03/042247
- US-A1- 2004 180 386
- ROQUE-NAVARRO LOURDES ET AL: "Humanization of predicted T-cell epitopes reduces the immunogenicity of chimeric antibodies: New evidence supporting a simple method." HYBRIDOMA AND HYBRIDOMICS, vol. 22, no. 4, August 2003 (2003-08), pages 245-257, XP002397469 ISSN: 1536-8599
- PENICHET MANUEL L ET AL: "Design and engineering human forms of monoclonal antibodies" DRUG DEVELOPMENT RESEARCH, vol. 61, no. 3, March 2004 (2004-03), pages 121-136, XP002397470 ISSN: 0272-4391
- DE GROOT A S ET AL: "DE-IMMUNIZATION OF THERAPEUTIC PROTEINS BY T-CELL EPITOPE MODIFICATION", DEVELOPMENTS IN BIOLOGICALS, KARGER, BASEL, vol. 122, 1 January 2005 (2005-01-01), pages 171-194, XP008074262, ISSN: 1424-6074
- BASELGA J: "PHASE I AND II CLINICAL TRIALS OF TRASTUZUMAB", ANNALS OF ONCOLOGY, KLUWER, DORDRECHT, NL, vol. 12, no. SUPPL. 01, 1 January 2001 (2001-01-01), pages S49-S55, XP009063466, ISSN: 0923-7534, DOI: 10.1023/A:1011167909059

## Description

The present invention relates to methods for screening composite antibody variable regions or antigen-binding fragments, to avoid helper T cell epitopes. The methods comprise screening libraries of genes encoding composite antibody variable regions. Such methods allow for generation of antibodies and proteins for use as pharmaceutical agents in humans or *as in vivo* diagnostic agents.

The last 20 years has seen great advances in the generation of recombinant monoclonal antibodies for use as potential pharmaceuticals in man. The techniques of chimerization, humanization and human antibody cloning either by phage display or transgenic mice have provided antibodies which are generally well tolerated when administered to man with less immunogenicity than with non-human monoclonal antibodies. However, several antibodies generated by these techniques have been shown to elicit immunogenicity in patients even where the genetic origins of such antibodies are human. For example, the human antibody Humira®, elicits immunogenicity in 12% of rheumatoid arthritis patients and the humanized antibody CAMPATH® elicits immunogenicity in about 50% of patients. Such induction of immunogenicity is likely to result from the presence, within the antibody variable region, of tracts of non-self amino acid sequences which, in some cases, can create T cell epitopes which induce T cell responses resulting in immunogenicity. There is therefore a need for improved techniques and antibody compositions which have a high human origin but which avoid, as much as possible, creation of sequences which might induce T cell responses.

The present invention provides screening whereby composite antibody variable regions or antigen-binding fragments thereof which combine two or more peptides of amino acid sequence 2 to 31 amino acids long are screened.

Thus, in a first aspect, the present invention provides a method for screening a composite antibody variable region or antigen-binding fragment to avoid helper T cell epitopes, wherein the method comprises the following steps:
Generating a library of genesencoding composite antibody variable regions or antigen-binding fragments derived from multiple peptides of amino acid sequence of 2 to 31 amino acids long from other antibodies or antigen-binding fragments, wherein the two or more peptides are neither whole CDRs nor whole framework regions;
Screening the antibody variable region or antigen-binding fragment to avoid helper T cell epitopes; and
Expressing and screening the library of antibody variable regions or antigen-binding fragments for binding to one or more antigens of interest.

In the context of the present invention, the peptides are contiguous amino acid sequence found within an antibody molecule, such being from 2 to 31 amino acids long where such peptides are neither whole CDRs nor whole framework regions. For therapeutic use, composite antibody variable regions will typically combine two or more peptides of amino acid sequence from different human antibodies within the composite variable regions. In particular, composite antibody heavy and light chain variable regions (VH and VL respectively) where each VH and VL is composed entirely of segments of sequence from two or more human antibody variable regions and where typically each composite VH and VL includes segments of human variable region sequence positions corresponding to their positions in the source human antibody VHs and VLs, for example amino acids 1 to 10 in the composite VH sequence will derive from amino acids 1 to 10 in a human antibody. Alternatively, segments of human VH or VL sequence in the composite antibody variable region may be positioned at any sequence location irrespective of the sequence position in the source human antibody VH or VL. The source human antibody VHs and VLs will be any existing human antibody variable region amino acid sequence, for example as provided in databases of human monoclonal antibody variable region sequences, and may include sequences from affinity-matured antibodies with variable region somatic mutations and other variations differing from germ-line, sequences from germ-line variable regions, sequences from artificially constructed antibody variable regions created from segments of sequence from antibodies of the species such as antibodies with a set of fixed variable region frameworks but with variable CDRs, sequences selected from human antibody libraries such as phage display libraries, and sequences of human antibodies derived from transgenic animals expressing genes encoding human antibodies or antibody fragments.

Human T cell epitopes in the context of the present invention are amino acid sequences which can bind to human MHC class II molecules and, through presentation to CD4⁺ T cells, induce a helper T cell response. Human VH and VL sequence segments and combinations of segments can be chosen which limit or avoid T cell epitopes in the final composite antibody variable region. This can be achieved by use of segments which do not contain T cell epitopes, such as from human germ-line sequences, and by joining of adjacent segments to create a new sequence which does not contain T cell epitopes, for example by creation of a non-MHC binding sequence at the junction of two segments, by creation of another human germ-line sequence, or by creation of a sequence which does not induce a helper T cell response despite a non-germ-line sequence.

As discussed herein, the present invention provides methods for screening of composite antibody variable regions. Typically, such methods comprise the steps;
(1) preparing antibody variable region genes by combining segments of amino acid sequence from a range of other antibody variable regions in order to generate a library of different variable region genes
(2) cloning the library of antibody variable region genes into an expression vector
(3) screening the library of antibody variable regions and recovering members of the library with desirable properties

In a first preferred method 'A' of the present invention, a library of composite human antibody regions is generated and screened for antibodies with desirable properties such as binding to a specific antigen. This method involves 6 steps as follows;
(1) design of composite VH and VL genes
(2) cloning of composite VH and VL genes
(3) expression of composite VH and VL genes
(4) screening and selection of composite antibodies with desirable properties
(5) optimisation of lead composite antibodies
(6) avoidance of T cell epitopes

For step (1), the library of composite VH and VL sequences are designed by selecting segments of VH and VL sequence from known human V region sequences such as those available in the Kabat antibody database (www.bioinf.org.uk/abs/simkab.html), the NCBI database (www.ncbi.nlm.nih.gov) and from protein databases, such as UniProt (www. ebi.uniprot.org) and PRF/SEQDB (www.prf.or.jp). In addition, these can be supplemented by collection of human VH and VL sequences by direct sequencing of amplified VH and VL mRNA from one or more individual donors. Various combinations of sequence segments can be considered for design of VH and VL genes. One method used is to fix the length of the composite VH and VL sequences and to design these using fixed length sequence segments from corresponding Kabat numbering positions in different human V regions.

For example, the library would comprise VH and VL regions of 121 and 107 amino acids respectively and would include, for example, an assortment of different segments for VH amino acids 1-27 using Kabat numbering. For VH with CDRs corresponding to Kabat numbering CDR1:30-35, CDR2:50-66 and CDR3:95-106, sequence segments for the following Kabat positions are used as one option: 1-27, 28-31, 32-36, 37-42, 43-50, 51-56, 57-60, 61-63, 64-69, 70-82a, 82b-96, 97-98, 99-101, 102-117. For VL with CDRs corresponding to Kabat numbering CDR1:24-34, CDR2: 50-56, CDR3:89-97, sequence segments for the following Kabat positions are used as one option: 1-22, 23-27, 28-30, 31-33, 34-35, 36-47, 48-52, 53-55, 56-59, 60-87, 88-92, 93-94, 95-107. Therefore, in this example, composite VHs are composed of 14 human segments and composite VLs are composed of 13 human segments. In practice, a computer program is used to generate combinations of these segments. Preferably, the program includes an algorithm to avoid non-preferred combinations of certain segments which might, for example, avoid certain canonical structures of CDRs or which might disrupt VH and/or VL folding or VH/VL interaction. As an optional addition, the program could include an algorithm to limit the number of T cell epitopes formed by the combination of sequence segments (see *in silico* methods in step (6) below)..

For step (2), having designed a library of composite human sequences, composite VH and VL genes are then generated preferably using synthetic oligonucleotides. Typically, synthetic oligonucleotides encoding longer segments of variable region sequence will be ligated to a mixture of oligonucleotides which encode two or more consecutive segments of variable region sequence. Alternatively, composite variable regions could be assembled by other methods such as overlapping PCR or other amplification techniques using existing human VH and VL genes as templates. For example, using PCR, small segments of variable regions can be amplified separately and then joined by overlapping PCR reactions.

In other methods, mixed synthetic oligonucleotides can be produced to create a range of sequence segments preferably using doping methods to enrich for sequences encoding specific variable region segments. Composite human VH and VL genes with extensive variability of human variable region segment representation can be assembled in many ways using techniques known to those skilled in the art such as those described in Molecular Cloning: A Laboaratory Manual; 3rd Ed., vols. 1-3 (2001) Cold Spring Harbor Laboratory Press and using standard PCR methods for immunoglobulins such as those described in Orlandi et al., Proc Natl Acad Sci U S A.,86 (1989) 3833-3837.

For step (3), once composite human VH and VL genes are generated, these can be cloned into a variety of expression vectors for production of either complete antibody molecules or antigen-binding fragments such as Fv's, Fab's, Fab2, SCAs, single domain antibodies (e.g. comprising VHs only) and multimeric derivatives of each of these. Alternatively, VH and VL genes can be fused to genes encoding other molecules to generate fusion proteins. Also included might be sequences encoding detectable markers such as poly-histidine tags at the C terminus of one chain of an Fv or Fab. Expression vectors include those for expression in mammalian cells, bacterial cells, bacteriophage, yeast, fungus and other micro-organisms. Such vectors also include those for expression *in vivo* from transgenic animals and those for expression *using in vitro* systems such as *in vitro* translation using ribosome preparations.

For step (4), screening of libraries of composite human antibodies is usually for binding to one or more specific antigens of interest. There are many screening methods known to those skilled in the art, the selection of which will depend on the form of expression of the composite human antibodies and the composition of the antibody molecules i.e. complete antibody or Fab, Fv, SCA, single domain antibody etc. In some cases where an existing antibody is available which binds to the antigen of interest, either VH or VL from this antibody may be combined with the composite human VL or VH respectively and tested for binding.

Screening methods will range from immobilising individual members of the library or pools of such members on a solid phase to immobilising the antigen of interest either individually or in pools. Where antibodies are immobilised, the antigen of interest is then added and is either detectable directly or indirectly by addition of one or more additional reagents. For example, if the antigen is a fusion protein or conjugate with an enzyme such as alkaline phosphatase, detection can be achieved by subsequent addition from a wide range of substrates which produce colour, fluorescence or chemiluminescent signals. Where antibody pools are immobilised in one location (e.g. the well of a microtitre dish) and a signal results from addition of antigen, this pool can then be dereplicated prior to rescreening of either individual members of the pool or smaller pools. Where the antigen of interest is immobilised, the composite antibody library may be screened in several ways ranging from addition of individual antibodies to the antigen of interest which is immobilised at a specific location, to addition of pools of antibody, to addition of the whole composite library and subsequent recovery of antibodies bound to the antigen of interest. In the last case, a common strategy is to immobilise antigen on a solid phase such as in a column or on beads, to add the library, to subsequently wash the solid phase for example with a low salt buffer (to detach loosely associated members of the library), and to then elute antibodies which bind to the antigen using, for example, a high salt buffer. Common formats for expression of members of the library for this purpose are phage display, yeast display, ribosome display and bead display, in each case where nucleic acid encoding composite VH and VL chains remains attached to the composite V region which binds to the antigen.

Screening methods will also include functional or biological tests which may be substituted for direct antigen binding tests where a functional or biological activity is measured such as *in vitro* tests involving cell growth, cell growth inhibition, cell differentiation or cell migration, or alternative *in vivo* tests involving measuring responses to the antigen at the level of the whole organism, for example changes in blood cell counts in a mouse or growth inhibition of a transplanted tumour.

For step (5), following selection of one or more "lead" composite human antibodies with desirable properties such as binding to an antigen of interest, optionally the properties of the lead antibody may be improved, for example by increasing affinity for binding to the antigen or fusing the antibody to an additional moiety. Increased affinity may be achieved by mutagenesis of composite variable region sequences in order to select for mutations in the selected composite V region sequences which increase or alter binding in a desirable way. The present invention includes novel methods for mutagenesis of variable region sequence by replacing one or more individual variable region sequence segments from the lead antibody with corresponding sequence segments from one or more human antibody sequences. In particular, segments overlapping with or within CDR region may be replaced by one or more alternative segments from other human antibodies including segments of different lengths. Within the scope of the invention, specific segments may be included from human antibodies with related properties to the selected lead antibody, for example from antibodies which bind to the same antigen, or from non-human antibodies with related properties, or from human antibodies with sequence segments which retain certain key amino acids which appear important for function in a non-human antibody with related sequence. One or more composite human antibodies subject to such mutagenesis can then be screened for improved properties.

For step (6), following selection of a lead composite human antibody, T cell epitopes are limited or avoided by, where required, exchanging variable region segments contributing to or encoding a T cell epitope with alternative segments which avoid T cell epitopes. Such T cell epitopes can be detected by a range of methods. For example, peptides corresponding to one or more loci in the composite V region sequence can be synthesised and tested in T cell assays to determine the presence of T cell epitopes. Typically such peptides will be 15 amino acids in length and, where it is desirable to test a longer contiguous variable region sequence, overlapping peptides from the sequence such as 15mers with 12 amino acid overlaps are used. For detection of T cell epitopes, a range of different T cell assays can be used for measurement of activation or proliferation of CD4+ T cells such as those measuring cytokine release, proliferation (for example, by uptake of 3H-thymidine), Ca2+ flux, surface marker expression, gene transcription etc.

Alternatively, overlapping peptides corresponding to the composite variable region sequences are analysed for binding to human MHC class II molecules either using in *vitro* methods or *in silico* methods, in each case to determine potential T cell epitopes i.e. MHC binding peptides which may induce a T cell response. *In silico* methods will include methods involving modelling of peptide-MHC class II binding interactions, methods involving identification of motifs common for binding to MHC class II and methods using databases of peptides or specific amino acids within peptides with known in vitro MHC binding properties. Other methods can be used such as producing longer peptides from composite variable region sequences or whole antibodies containing composite variable region sequences and testing these in T cell assays or in MHC binding assays, for example by testing for MHC-peptide tetramers, or by searching the proposed or constructed sequences in a database of known human T cell epitopes. Avoidance of T cell epitopes in composite human V regions can also be assisted by avoidance of MHC class II binding motifs or avoidance of particular amino acids which anchor the binding of peptides to MHC class II. In the preferred method for avoidance of T cell epitopes from one or more lead composite human antibodies, *in silico* methods are initially applied to analyse the composite human antibody variable regions for potential T cell epitopes and, where these are identified, new segments of human VH or VL sequence are introduced to avoid these epitopes and to avoid introduction of new T cell epitopes.

Following any such introduction of new human variable region segments and rescreening of such modified lead composite human antibodies for desirable properties, one or more final lead composite human variable region can then be further tested in human T cell assays either by testing overlapping peptides typically of 15 to 45 amino acids in length, for example 15mer peptides with 12 amino acid overlaps from the composite human variable region sequences (whole variable regions or parts thereof) or by testing whole composite human antibodies directly in human T cell assays. A final analysis using T cell assays for testing whole composite human antibody is preferred allowing for direct testing for T cell activation against the whole antibody.

In a second preferred method 'B' of the present invention, a library of composite human antibodies is generated to include desirable amino acids from one or more reference antibodies with desirable properties. This method involves 7 steps as follows;
(1) sequence analysis of one or more reference antibodies
(2) design of composite VH and VL genes
(3) avoidance of T cell epitopes
(4) cloning of composite VH and VL genes
(5) expression of composite VH and VL genes
(6) screening and selection of composite antibodies with desirable properties
(7) optimisation of lead composite antibodies including avoidance of T cell epitopes

In step (1), typical reference antibodies will be rodent, especially mouse, with properties and/or binding specificities which are desirable in a human form of antibody. Where one or more reference antibody variable region sequences are available, these are analysed to determine sequences of the CDRs and to identify amino acids which might be important for the desirable properties of the antibody such as binding specificity. For a reference antibody, such analysis is performed, for example, by alignment of the reference variable region sequences with other sequences of the same species and also, if the reference antibody is non-human, human V region sequences. Such aligments are performed, for example, using the program CLUSTAL (Thompson et al., Nucleic Acids Res. 22 (1994) p4673-80). Such alignments can identify unusual or rare amino acids in the V region of the reference antibody and homologous variable region families. In addition, conserved variable region structures such as canonical structures of the CDRs can be identified using, for example, the Protein Data Bank (Berman et al.: The Protein Data Bank, Nucleic Acids Research, 28 (2000) 235-242). In addition, the reference antibody variable regions can be modelled, where a structure is not known, using modelling software such as MODELLER (Sali and Blundell, J. Mol. Biol. 234 (1993) p779-815) and, in some cases, models of antibody-antigen interactions can be generated. Such analyses of the reference antibody variable regions are used to guide on selection of segments of human variable region sequence for the composite human antibody.

For step (2), having determined amino acids which might be important for the desirable properties of the composite human antibody, segments of human variable region sequences are then selected to include some or all of these amino acids. A library of composite human variable region sequences is thereby designed including selected segments with typically one or more alternative human variable region segments at particular loci where the effect of such segments on properties of the composite human antibody is uncertain. Such composite human antibody sequences can be further analysed as with the reference antibody(s) by alignment with other human antibody sequences and conserved structures and, in addition, further modelling of the structure of composite human antibody variable regions can be undertaken in order to refine, as required, the combinations of human variable region segments used in the composite human antibodies to avoid defects in protein structure, intermolecular and intramolecular interactions within composite variable regions, and incorrect structural orientations of important amino acids.

For step (3), as an additional criteria for selection of segments, those segments or combinations of segments which limit or avoid T cell epitopes in the final composite human variable regions are selected. T cell epitopes are analysed by the methods described in method A, step (6) above using *in silico* or *in vitro* methods, preferably by use of *in silico* methods at the stage of designing composite human variable region sequences.

For step (4), having designed a library of composite human sequences, composite VH and VL genes are then generated preferably using synthetic oligonucleotides. Typically, synthetic oligonucleotides encoding longer segments of variable region sequence will ligated to a mixture of oligonucleotides which encode alternative segments of sequence to generate different members of the library of composite human variable regions. Alternatively, each member of the library of composite human V regions will be generated separately using oligonucleotides encoding the sequence of the specific human variable region. Alternatively, composite variable regions can be assembled by other methods such as overlapping PCR or other amplification techniques using existing human VH and VL genes as templates or using one or more reference antibody variable region genes as template.

Steps (5) and (6) for method B are as described in method A, steps (4) and (5).

Step (7) will be employed as in method A, step (6) where further avoidance of T cell epitopes is required in the lead composite human antibody(s). A final analysis using T cell assays for testing whole composite human antibody is preferred allowing for direct testing for T cell activation from the whole antibody.

It will be understood to those skilled in the art that, in addition to methods A and B, there will be other methods for screening and testing composite human antibody variable regions and for optimising their properties. The antibody variable regions which have been screened according to the invention are new and, as a result of the human origin of the variable regions and the avoidance of helper T cell epitopes, should be less immunogenic in humans than other antibody variable regions containing non-human sequences. It will be understood by those skilled in the art that the object of lower immunogenicity may be achieved using less preferred composite antibodies containing variable regions without all human sequence segments, for example composite human antibody variable regions including segments at sequence positions in the composite antibody variable region different from their sequence positions in the source human antibody, composite antibody variable regions with only partial incorporation of segments of human variable region sequence, composite antibody variable regions with segments of non-human sequence, or composite antibody variable regions with human sequence which has been mutated, for example to increase binding affinity to an antigen or to avoid a T cell eptiope.

It will be understood by those skilled in the art that, in addition to human, composite antibody variable regions with variable region segments derived from other species either wholly or in part can be generated and should be considered within the scope of the invention. For example, for studies in mice, composite mouse antibody variable regions can be generated comprising variable region sequence segments wholly or partly of mouse origin.

As discussed herein, the composite antibody variable regions described herein are useful in treating disease and exhibit less immunogenicity. Thus, they can be used in a pharmaceutical formulation comprising a composite antibody variable region or antigen-binding fragment as defined herein, optionally together with one or more pharmaceutically acceptable excipients, carriers or diluents.

These compositions may be presented in unit dose forms containing a predetermined amount of each active ingredient per dose. Such a unit may be adapted to provide 5-100mg/day of the compound, preferably either 5-15mg/day, 10-30mg/day, 25-50mg/day 40-80mg/day or 60-100mg/day. For compounds of formula I, doses in the range 100-1000mg/day are provided, preferably either 100-400mg/day, 300-600mg/day or 500-1000mg/day. Such doses can be provided in a single dose or as a number of discrete doses. The ultimate dose will of course depend on the condition being treated, the route of administration and the age, weight and condition of the patient and will be at the doctor's discretion.

The compositions may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols or oils.

For applications to the eye or other external tissues, for example the mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administration to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

Pharmaceutical formulations adapted for rectal administration may be presented as suppositories or enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists which may be generated by means of various types of metered dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

Preferred unit dosage formulations are those containing a daily dose or sub-dose, as herein above recited, or an appropriate fraction thereof, of an active ingredient.

It should be understood that in addition to the ingredients particularly mentioned above, the formulations may also include other agents conventional in the art having regard to the type of formulation in question, for example those suitable for oral administration may include flavouring agents.

The following examples should not be considered limiting for the scope of the invention. The figures and tables relate to the examples below and are as follows;
Figure 1/2 - Sequence of VH (figure 1) and VL (figure 2) genes used for Composite Human anti-HER2 antibody
Figure 3 - Inhibition of proliferation of human SK-BR-3 cells after 8 days incubation with chimeric 4D5 IgG1/kappa, Composite Human anti-HER2 antibody and epitope avoided anti-HER2 "EACHAB" with chimeric anti-IgE control (see example 4)
Figures 4/5 - Sequence of VH (figure 4) and VL (figure 5) genes used for Composite Human anti-Lewis Y antibody
Figures 6/7 - Sequence of VH (figure 6) and VL (figure 7) genes used for Composite Human anti-human IgE antibody
Figure 8 - Sequence of VH and VL genes used for Composite Mouse anti-human TNFα antibody including avoidance of human T cell epitopes
Figure 9 - ELISA for binding to human TNFα by Composite Mouse and chimeric anti-human TNFα antibody
Figure 10 - V region sequences of anti-TNFα antibody A2
Figure 11 - Sequences of composite human anti-TNFα VH variants
Figure 12 - Sequences of composite human anti-TNFα VL variants
Figures 13/14 - Oligonucleotides for construction of chimeric mouse:human anti-TNFα VH (figure 13) and VL (figure 14)
Figures 15/16 - Oligonucleotides for construction of primary composite human anti-TNFα VH (figure 15; corresponding to SEQ ID No. 3 figure 11) and VL (figure 16; corresponding to SEQ ID No. 4 figure 12)
Figures 17- Oligonucleotides for construction of secondary composite human anti-TNFα VH and VL variants
Figure 18 - WEHI-164 protection Assay for composite human anti-TNFα antibodies
Figure 19 - Time-Course human T cell assay of lead composite human anti-TNFα

Tables 1-3 - CDRs used in Composite Human antibody scFv library comprising 186 x 9 residue-long VH CDR3s (table 1), 77 x 8 residue-long VL CDR3s (table 2), and 153 x 10 residue-long VL CDR3s (table 3).

Table 4 - Human sequence segments used for primary composite human anti-TNFα VH and VL variants

Table 5 - Activity of composite human anti-TNFα variants

### Example 1 - Construction of Composite Human Anti-HER2 Antibody

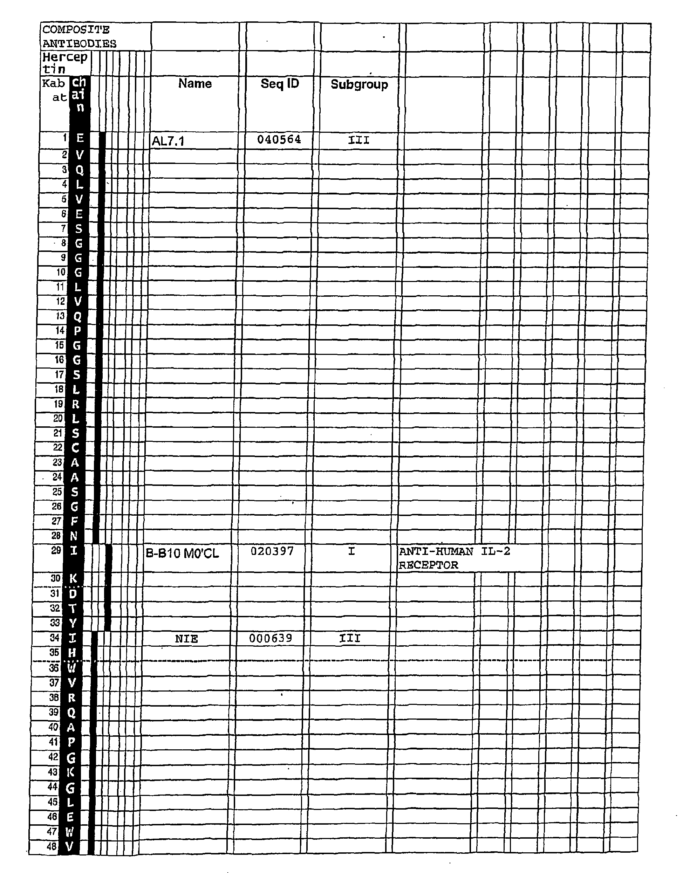

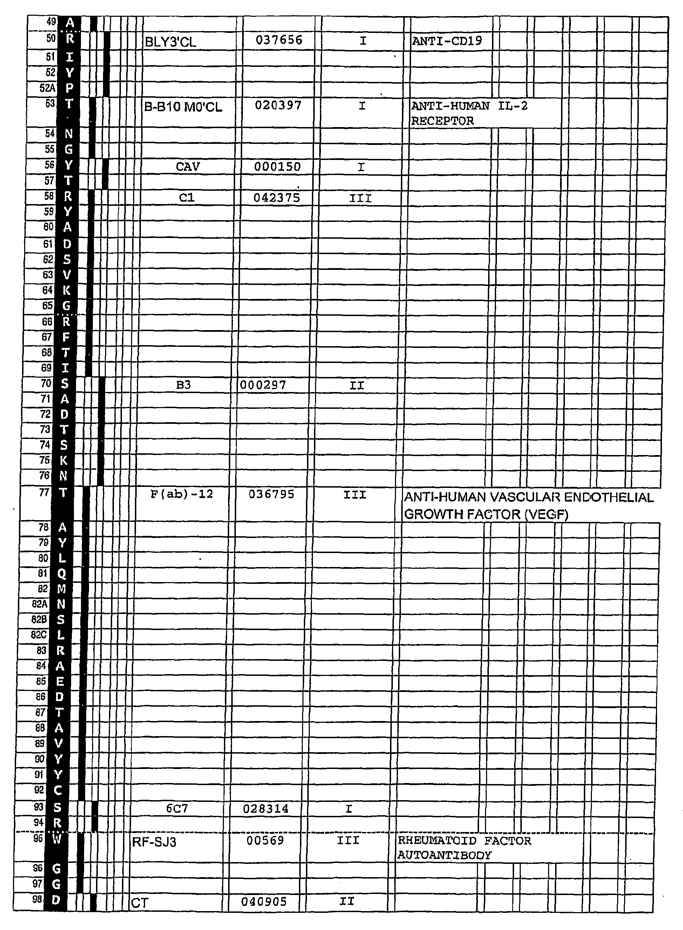

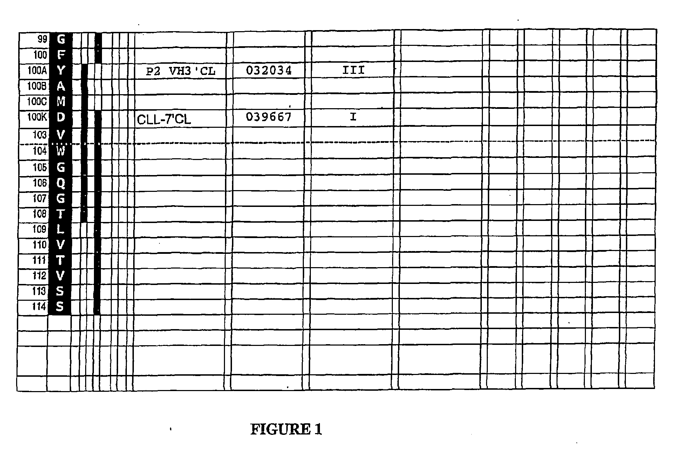

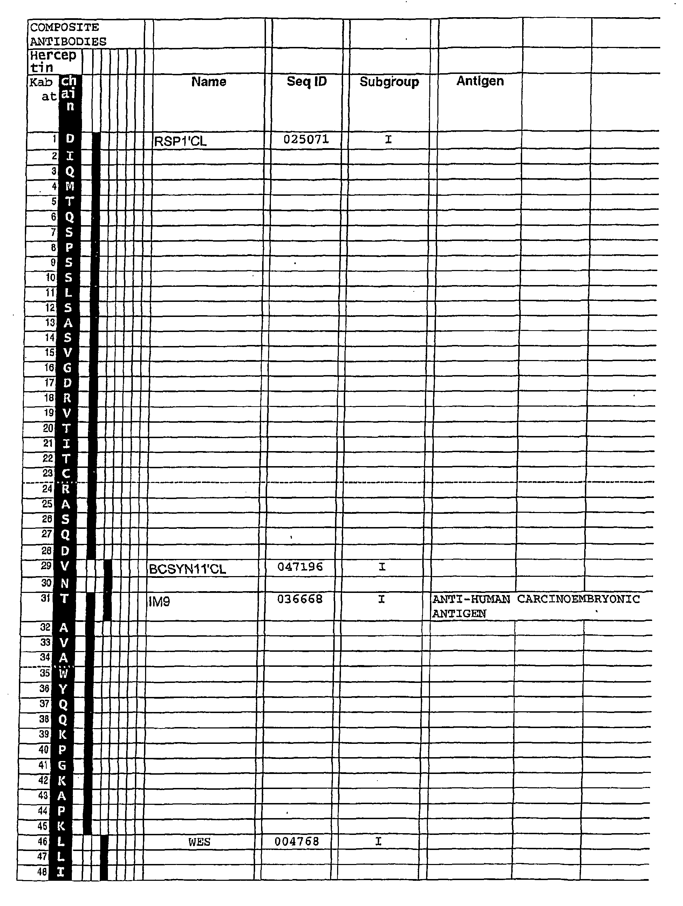

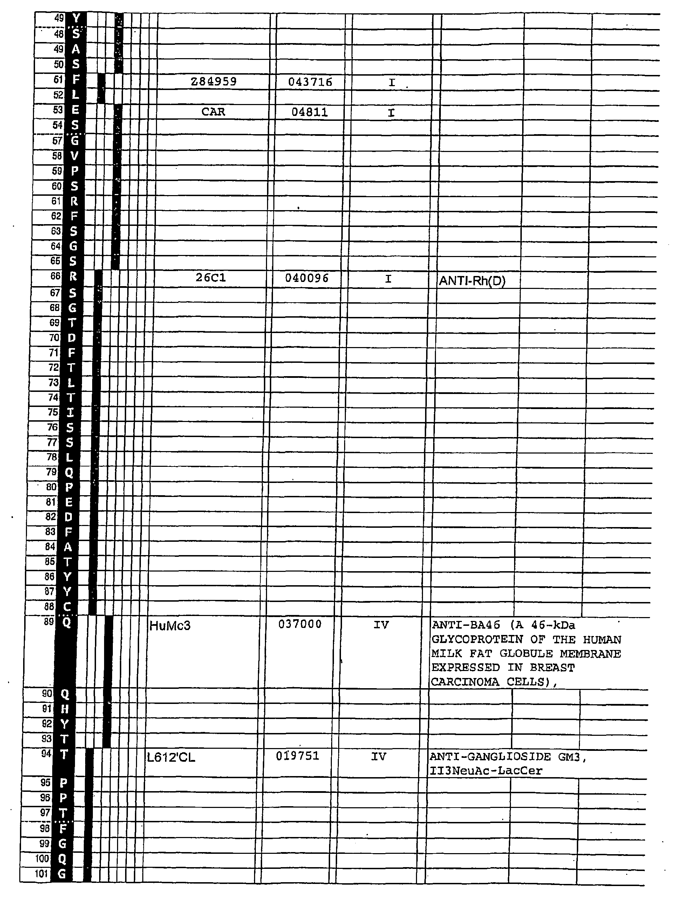

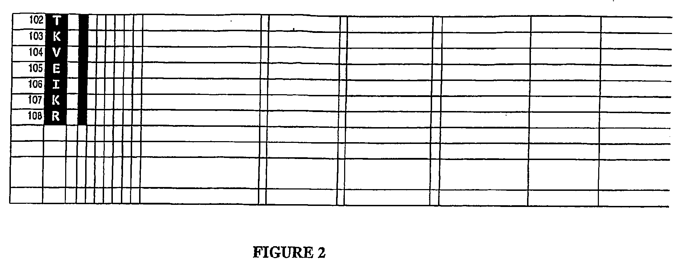

For creation of a human variable region sequence segment library, amino acid sequences from a range of human immunoglobulins were collected into a single database comprising the *in silico* human variable region sequence library including heavy (VH) and light (VL) chain variable region sequences. Sources of sequences included the NCBI Igblast database (www.ncbi.nih.gov), Kabat databases (Kabat et al., Sequences of Proteins of Immunological Interest, NIH publication 91-3242, 5th ed. (1991) (and later updates)), Vbase (www.mrc-cpe.cam.ac.uk/imt.doc), Genbank (Benson et al., Nucl. Acids Res. 25 (1997) p1-6 or via www.bioinf.org.uk/abs) databases. The reference antibody variable region sequences used was a humanised anti-HER2 antibody known as Herceptin® (Carter et al., Proc. Nat. Acad. Sci. USA, vol 89 (1992) p4285, US5821337). Segments from the *in silico* human variable region sequence library were selected for identity to the corresponding amino acids in the Herceptin® variable region sequence and combined to produce the composite human VH and VL sequences as shown in figures 1 and 2 respectively.

Recombinant DNA techniques were performed using methods well known in the art and, as appropriate, supplier instructions for use of enzymes used in these methods. Sources of general methods included Molecular Cloning, A Laboratory Manual, 3rd edition, vols 1-3, eds. Sambrook and Russel (2001) Cold Spring Harbor Laboratory Press, and Current Protocols in Molecular Biology, ed. Ausubel, John Wiley and Sons. Detailed laboratory methods are also described in example 7 below. Composite human VH and VL sequences corresponding to Herceptin® were created using, for each chain, eight synthetic oligonucleotides of 30-60 amino acids in length encoding the entire composite human VH and VL sequences. In parallel, as a control reagent, a chimeric form of the mouse monoclonal antibody 4D5 (Hudziak et al., Mol. Cell. Biol., (March 1989) p1165-1172)), was also created using eight synthetic oligonucleotides per chain. Separate VH and VL oligonucleotides were first phosphorylated, mixed at equal molar ratios, heated to 94oC for 5 min in a thermal cycler followed by cooling to 65oC and incubation at 65oC for 2 min. Incubations were then continued at 45oC for 2 min., 35oC for 2 min., 25oC for 2 min and 4oC for 30 min. Oligonucleotides were then ligated using T4 DNA ligase (Life Technologies, Paisley UK) at 14oC for 18 hours.

To each of the VH and VL oligonucleotide mixtures, additional oligonucleotides encoding a 5' flanking sequence, including a Kozak sequence, the leader signal peptide sequence and the leader intron, and 3' flanking sequence, including the splice site and intron sequence, were added and annealed as above. The Composite Human V_{H} and V_{K} and the 4D5 expression cassettes produced were cloned as HindIII to BamHI fragments into the plasmid vector pUC19 and the entire DNA sequence was confirmed. These were transferred to the expression vectors pSVgpt and pSVhyg which include human IgG1 (V_{H}) or Kappa (V_{K}) constant regions respectively and markers for selection in mammalian cells. The DNA sequence was confirmed to be correct for the Composite Human V_{H} and V_{K} and 4D5 V_{H} and V_{K} in the expression vectors.

The host cell line for antibody expression was NS0, a non-immunoglobulin producing mouse myeloma, obtained from the European Collection of Animal Cell Cultures, Porton, UK (ECACC No 85110503). The heavy and light chain expression vectors were co-transfected into NS0 cells by electroporation. Colonies expressing the gpt gene were selected in Dulbecco's Modified Eagle's Medium (DMEM) supplemented with 10% foetal bovine serum, 0.8 g/ml mycophenolic acid and 250 g/ml xanthine. Transfected cell clones were screened for production of human antibody by ELISA for human IgG. Cell lines secreting antibody were expanded and the highest producers selected and frozen down in liquid nitrogen. The modified antibodies were purified using Prosep -A (Bioprocessing Ltd, Northumberland, UK). The concentration was determined by ELISA for human IgG antibody.

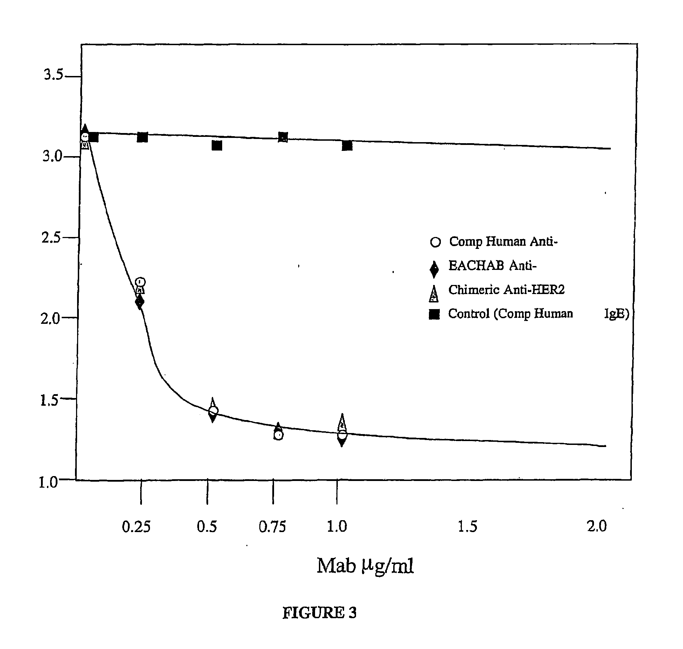

The Composite Human antibody and chimeric 4D5 antibodies were tested for inhibition of proliferation of the HER2+ human breast tumour cell line SK-BR-3 in conjunction with a negative control non-Her-2 binding human IgG1 / Kappa antibody exactly as described by Hudziak et al. (ibid). The results (figure 3) show that Composite Human antibody and the chimeric 4D5 antibodies have equivalent potency in inhibiting growth of SK-BR-3 cells. Figure 3 also shows data for an alternative "epitope avoided" Composite Human antibody produced as below.

In order to test the epitope avoidance option in the invention, the sequences of the Composite Human heavy and light chain variable regions were analysed for non-self human MHC class II binders using Peptide Threading
(www.csd.abdn.ac.uk/ gjlk/MHC-thread). This software predicts favourable interactions between amino acid side chains of the peptide and specific binding pockets within the MHC class II binding groove. All overlapping 13mers from the Composite Human heavy and light chain variable sequences were threaded through a database of MHC class II allotypes and scored based on their fit and interactions with the MHC class II molecules. Peptides predicted to bind MHC class II were 13mers beginning at residues 16 and 67 in VH, and 9 and 44 in VL. As a result, new segments of the human variable region sequence library were chosen instead of those used in the Composite Human sequences of figure 1 in order to introduce the amino acid changes VH 18L-A / 69I-G; VL 11L-A, 46L-A. A corresponding "epitope avoided" Composite Human antibody ("EACHAB" = Epitope Avoided Composite Human AntiBody) was made by substituting some of the oligonucleotides used to make the antibody corresponding to the sequence in figure 1 and the EACHAB was made as in the method described above and tested to show inhibition of SK-BR-5 proliferation equivalent to the standard Composite Human antibody (figure 3). This data shows that Composite Human antibodies can be successfully constructed with equal potency to a control chimeric anti-Her-2 antibody and that an EACHAB version of the Composite Human antibody can be generated without loss of potency.

### Example 2 - Immunogenicity of Composite Human Anti-HER2 Antibody

T cell proliferation assays were carried out to compare the immunogenicity of the Composite Human anti-HER2 antibody, the EACHAB variant and the chimaeric 4D5 antibody (see example 1). These antibodies were prepared from NS0 cells grown in serum-free, animal derived component-free, protein-free medium, HyClone HyQ®ADCF-Mab™ (Hyclone Cat No: Cat no: SH30349) supplemented with HyQ®LS1000 Lipid Supplement (Hyclone Cat No: SH30554) and sodium pyruvate (Gibco Cat No: 11360-039). After buffer exchange into 50mM MES pH6 on a Sephadex G25 (PD10 column), the antibodies were each passed through a cation exchange column (Mono-S 10/10) and eluted with a sodium chloride gradient (0 to 0.5M). The antibody containing fractions were then applied to a Superdex 200 preparative column (XK16/60) run in PBS. Peak fractions were pooled and stored at 4 C. The antibody concentrations were determined by ELISA for human IgG. Immunogenicity analysis was performed using PBMCs (peripheral blood mononuclear cells) that were isolated from healthy human donor blood and cryopreserved in liquid nitrogen. Each donor was tissue-typed using an Allset™ PCR based tissue-typing kit (Dynal, Wirral, UK) and 20 healthy donors were selected according to individual MHC haplotypes. 2ml bulk cultures containing 4x10⁶ PBMC in AIM V (Invitrogen, Paisley, UK) were incubated in a 24 well tissue-culture plate with test peptides (5 M final concentration) and proliferation was assessed on days 5, 6, 7, and 8 by gently resuspending the bulk cultures and transferring triplicate 100 1 samples of PBMC to a U-bottomed 96 well plate. Cultures were harvested onto glass fibre filter mats using a Tomtec Mach III plate harvester (Receptor Technologies, UK) and counts per minute (cpm) values determined by scintillation counting using a Wallac Microbeta TriLux plate reader (using a paralux high efficiency counting protocol). For each test antibody, the stimulation index (SI) was calculated as the ratio of counts per minute (cpm) of the test antibody : cpm of the negative control with SI>2 considered a significant T cell epitope response. The results showed that the chimaeric 4D5 antibody induced significant proliferative responses on at least one of the four days of proliferation tested (SI greater than 2) in five of twenty healthy donors tested (25%), the Composite Human anti-HER2 antibody induced SI>2 in three of twenty donors (15%) whilst the EACHAB anti-HER2 antibody induced SI>2 in none of twenty donors (0%). These results indicated an order of immunogenicity of chimeric 4D5> Composite Human anti-HER2>EACHAB anti-HER2 with the latter showing no evidence of immunogenicity in any donor blood sample tested.

### Example 3 - Construction of Composite Human Anti-Lewis Y Antibody

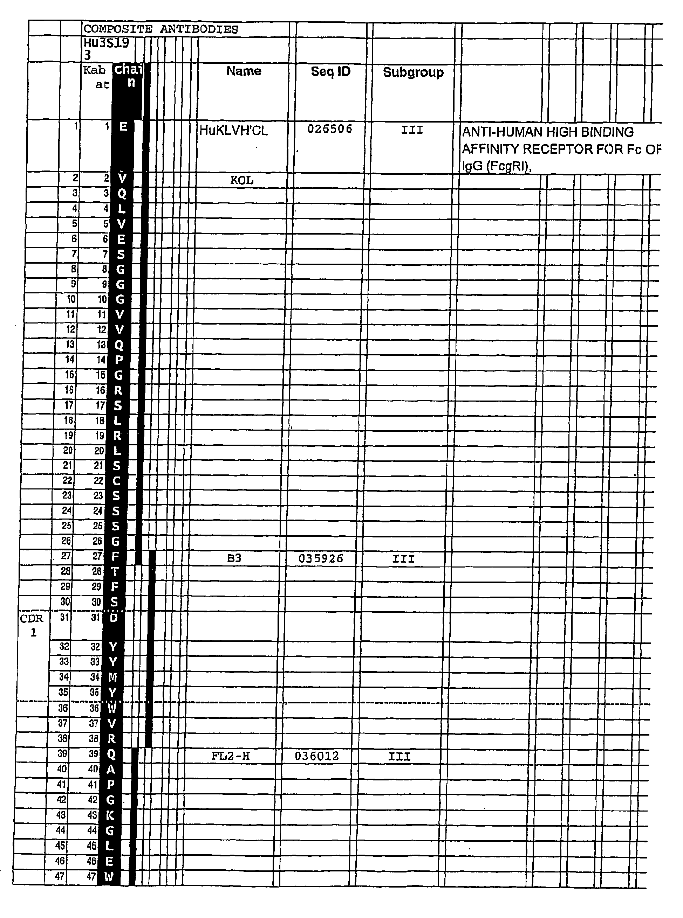

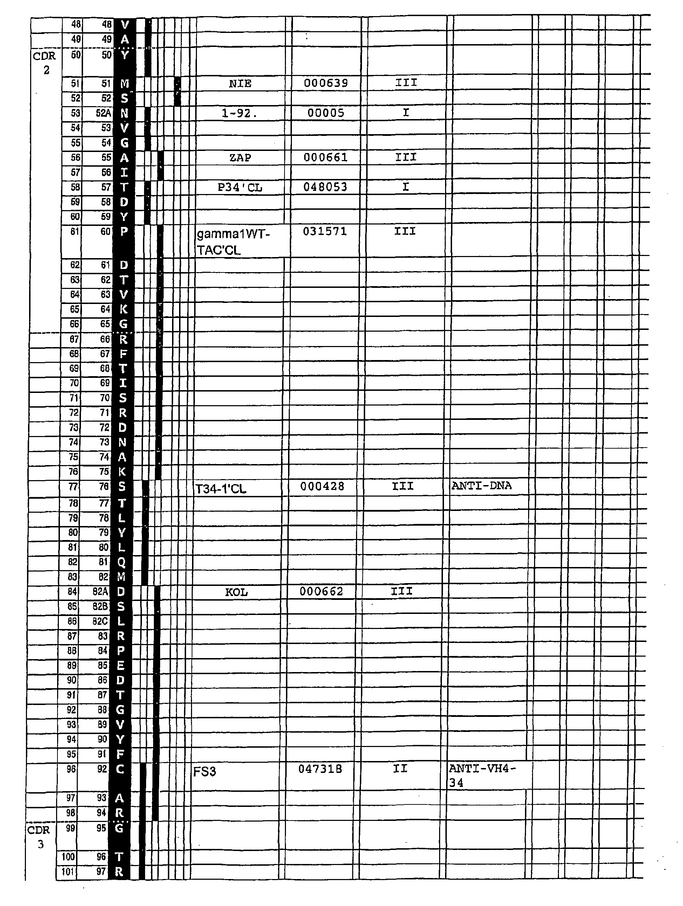

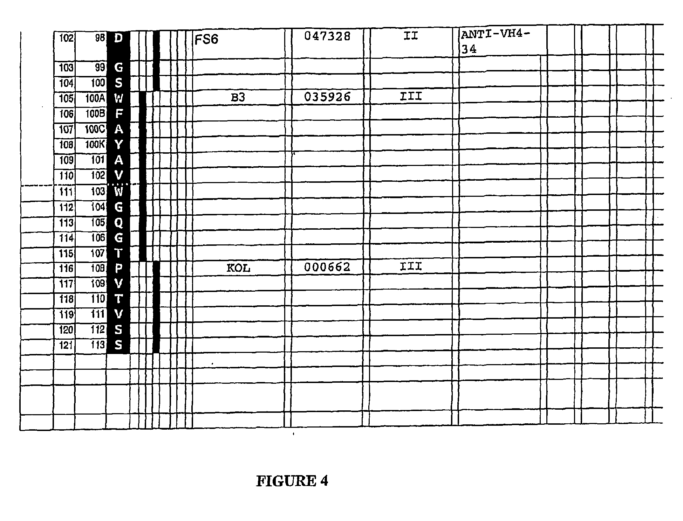

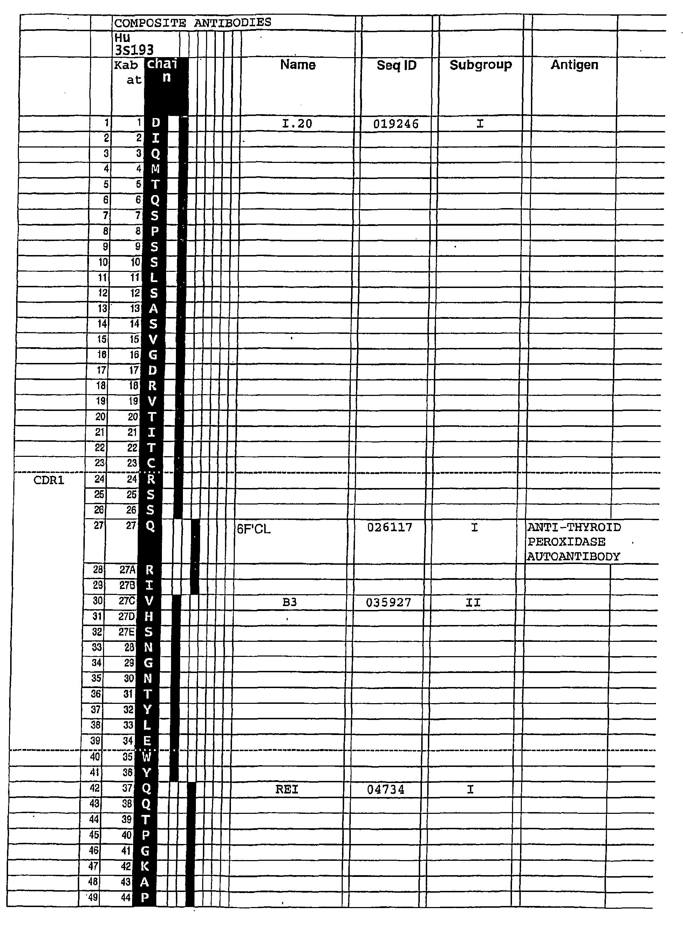

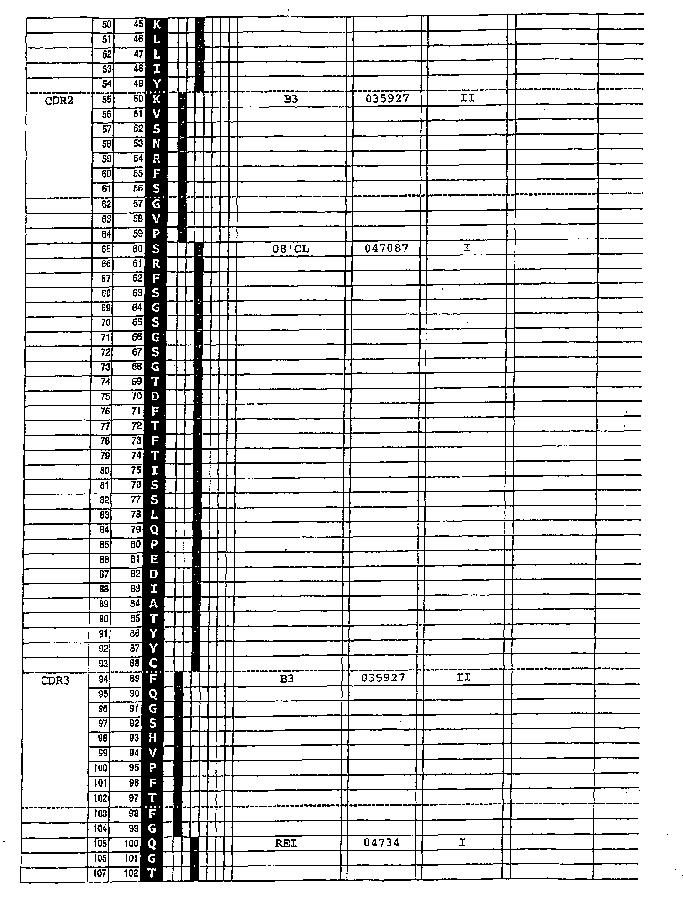

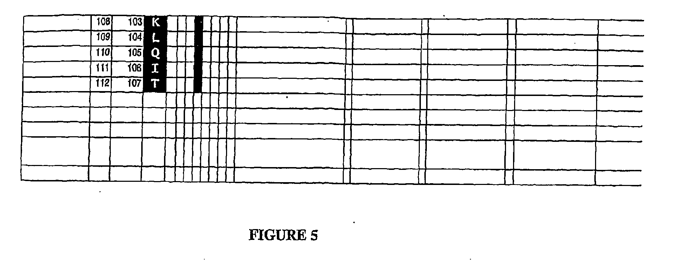

A Composite Human antibody specific for sialylated Lewis Y antigen was constructed as described in example 1 using, as the reference antibody variable region sequences, the humanised 3S193 antibody (Scott et al., Cancer Res., 60 (2000) p3254-3261, US5874060). Segments from the in silico human variable region sequence library were selected for identity to the corresponding amino acids in the humanised 3S 193 variable region sequence and combined to produce the Composite Human VH and VL sequences as shown in figures 4 and 5 respectively. In parallel, a reference chimeric anti-Lewis Y antibody was made from the reference V region sequences. Human IgG1 (V_{H}) and Kappa (V_{K}) constant regions were used on both the Composite Human anti-Lewis Y antibody and the chimeric reference antibody and antibodies were tested by ELISA against synthetic Lewis Y-HSA conjugate as described in US5874060. The data showed a minimum concentration of 0.1ug/ml chimeric antibody to give a binding signal in the assay compared to 0.15ug/ml Composite Human antibody which is consistent with the data of US5874060.

### Example 4 - Construction of Composite Human Anti-IgE Antibody

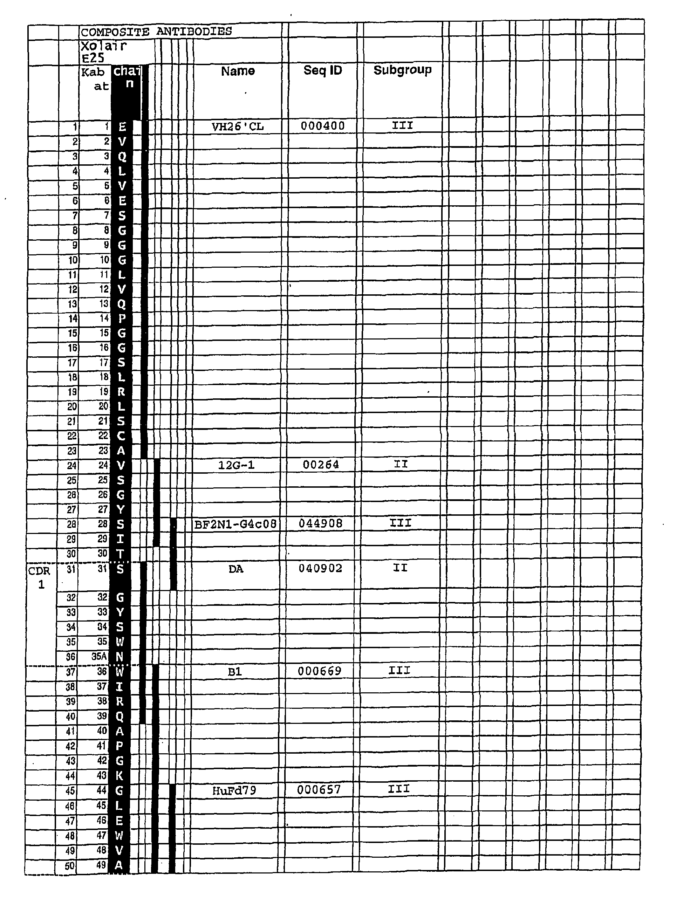

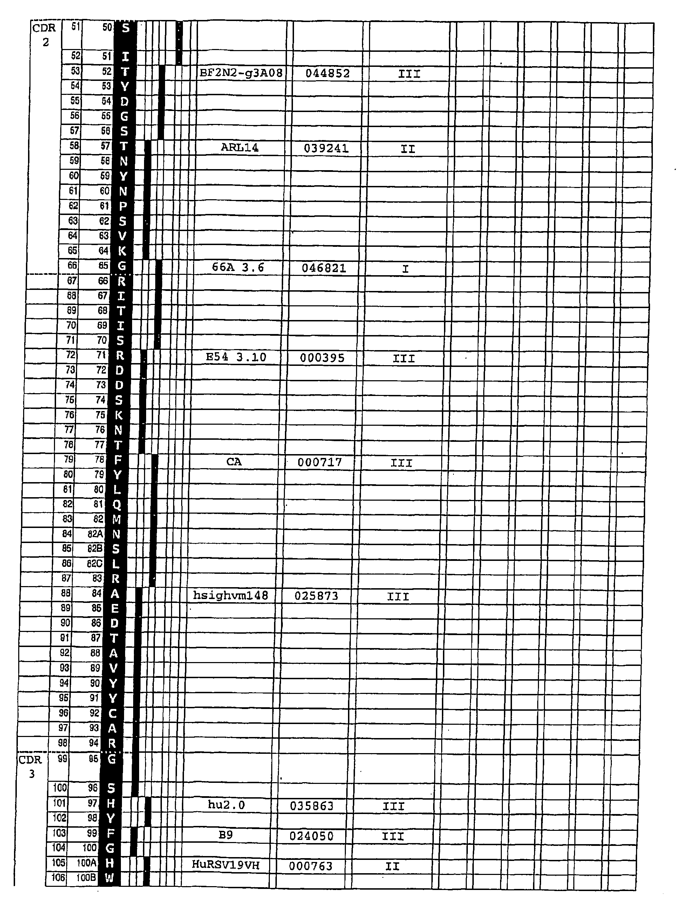

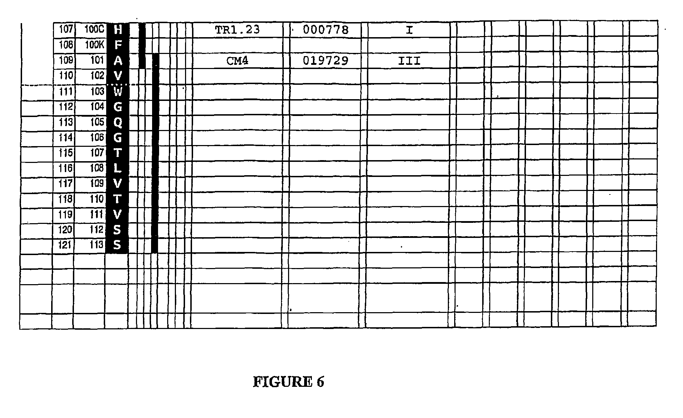

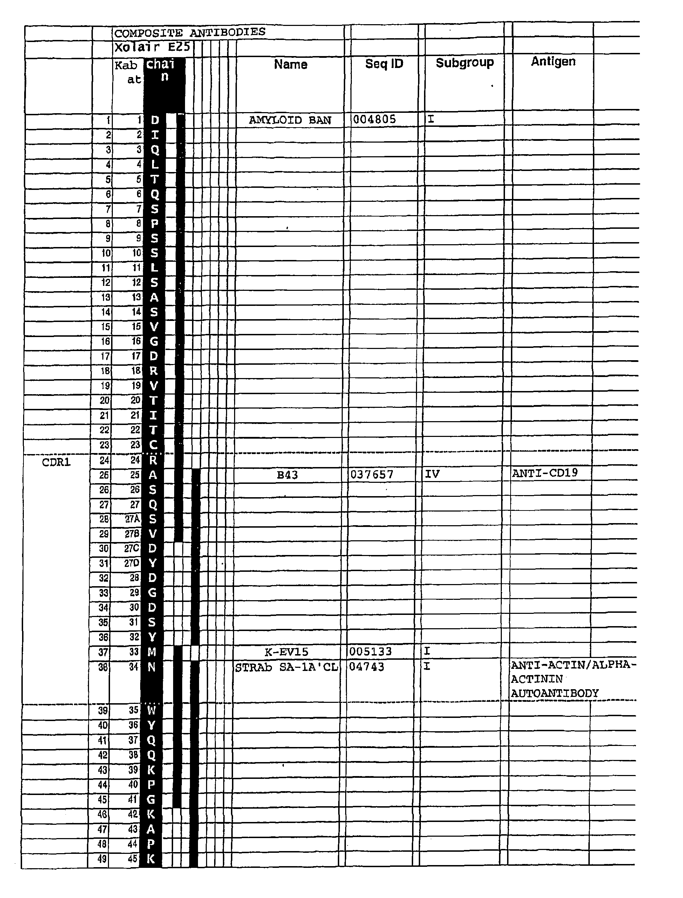

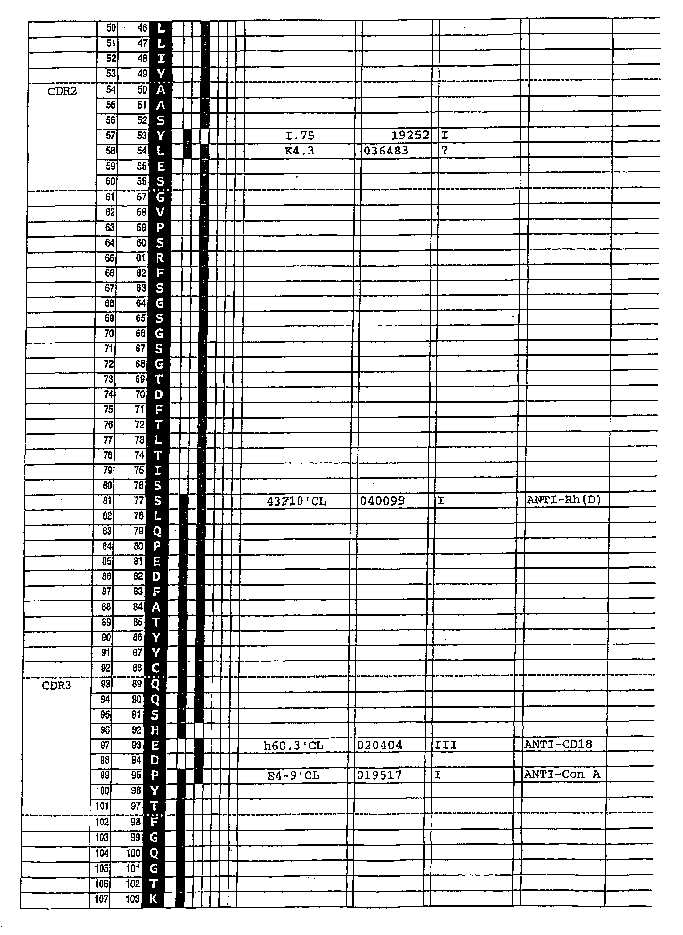

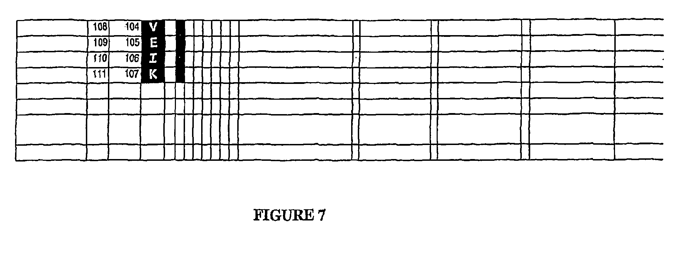

A Composite Human Anti-IgE antibody was constructed as described in example 1 using, as the reference antibody variable region sequences, the humanised anti-IgE antibody known as Xolair® (Presta et al., J. Immunol., 151(5) (1993) p2623-2632). Segments from the in silico human variable region sequence library were selected for identity to the corresponding amino acids in the Xolair® variable region sequence and combined to produce the Composite Human VH and VL sequences as shown in figures 6 and 7 respectively. In parallel, a reference chimeric anti-IgE antibody was made from the reference V region sequences. Human IgG1 (V_{H}) and Kappa (V_{K}) constant regions were used on both the Composite Human Anti-IgE antibody and the chimeric reference antibody.

The specificity of the Fabs was further characterized by surface plasmon resonance (BIAcore 2000, Biacore AB, Uppsala, Sweden). Recombinant human IgE Fab was produced as decscribed by Flicker et al., J. Immunol., 165 (2000) p3849-3859. Test antibodies were purified and immobilized onto flow cells of a CM chip using a NHS/EDC kit (Biacore) to obtain 2010 RU for chimeric anti-IgE and 2029 RU for Composite Human anti-IgE. 10 and 25nM recombinant human IgE Fab in Hepes-buffered saline (10 mM Hepes, 3.4 mM EDTA, 150 mM NaCl, 0.05% (v/v) surfactant P20, pH 7.4) was passed over the test antibodies at a flow rate of 5 µl/min for 10 minutes. The results showed that for both 10 and 25nM IgE Fab, an equivalent SPR (surface plasmon resonance) curve was detected for the chimeric anti-IgE and the Composite Human anti-IgE antibodies showing that the latter had successfully achieved binding efficiency equivalent to the reference anti-IgE antibody.

### Example 5 - Generation and Screening of Composite Human scFv Libraries

The strategy for initial construction of the human scFv (single-chain Fv) library was to construct seven consensus human VH and four consensus human VL (kappa) genes as detailed in Knappik et al., J. Mol. Biol., 296 (2000) 57-86 and to clone into these a large number of VH and VL CDR3 segments from databases of human variable regions. This list of CDR3s is shown in table 1 for VH CDR3s, table 2 for VL CDR3s of 8 amino acids and table 3 for VL CDR3s of 10 amino acids. For the master VH and VL construction, 6 overlapping synthetic oligonucleotides encoding VH and VL up to the end of framework 3 were synthesised as detailed by Knappik et al., ibid, and subjected to recursive PCR (Prodromou and Pearl, Protein Engineering, 5 (1992) 827-829). These were ligated into EcoRV digested pZero-1 vector (Invitrogen, Paisley, UK). For addition of CH1 and C kappa, both initially with 4D5 CDR3s (Carter et al, Bio/Technology, 10 (1992) 163-167), the protocol of Knappik et al., ibid, was followed except that the VH-CH1 SapI-EcoRI and VL-C kappa NsiI-SphI fragments were both blunt-end cloned into EcoRV digested pZero-1.

**Table 1**

| # Name | H3 | Length-H3 | Subgroup (H ) |
|---|---|---|---|
| MUC1-1'CL | DFLSGYLDY | 9 | I |
| ALL1-1'CL | VRGSGSFDY | 9 | III |
| ALL7-1'CL | DRGGNYFDY | 9 | III |
| L36'CL | MYNWNFFDY | 9 | I |
| 5.M13'CL | AGLGMIFDY | 9 | I |
| Au2.1'CL | RGFNGQLIF | 9 | I |
| M71'CL | ALTGDAFDI | 9 | II |
| VH6.N1'CL | TKLDWYFDL | 9 | II |
| E55 6.X'CL | RYGGFYFDY | 9 | II |
| E55 6.11'CL | GYSNEGMDV | 9 | II |
| VH6.A5'CL | SWDGYSYIY | 9 | II |
| VH6-EX8'CL | QMGAEYFQH | 9 | III |
| E54 4.2'CL | DMSLDAFDI | 9 | II |
| RF-SJ4'CL | GSVGATLGE | 9 | II |
| 3.A290'CL | YGDYHYFDY | 9 | III |
| A95 | GVGSSGWDH | 9 | III |
| 60P2'CL | KGSLYYFDY | 9 | III |
| E55 3.6'CL | PNWNDAFDI | 9 | III |
| E55 3.16'CL | RGIPHAFDI | 9 | III |
| 333'CL | PPEVESLRS | 9 | III |
| 1H1'CL | PPEVESLRS | 9 | III |
| 126'CL | PPEVESLRS | 9 | III |
| 1B11'CL | PPEVESLRS | 9 | III |
| 115'CL | PPEVESLRS | 9 | III |
| 112'CL | PPEVESLRS | 9 | III |
| 2C12'CL | PPEVESLRS | 9 | III |
| 2A12'CL | PPEVESLRS | 9 | III |
| BUT | DLAAARLF? | 9 | III |
| KOL-based resh. | QGTIAGIRH | 9 | III |
| CAMPATH-9 | | | |
| L2E8'CL | EDYYYGMDV | 9 | III |
| s5D4'CL | DPINWYFDL | 9 | III |
| ss4'CL | DRAAGDRDY | 9 | III |
| P2-57'CL | HQMYSNSDY | 9 | I |
| HuHMFG1'CL | SYDFAWFAY | 9 | I |
| NEW-based resh. | QGTIAGIRH | 9 | II |
| CAMPATH-9 | | | |
| TR1.10'CL | VLGIIAADH | 9 | I |
| L3B2'CL | DLTGDAFDI | 9 | I |
| DAW | SCGSQYFDY | 9 | II |
| ss7'CL | LWNWDAFDI | 9 | I |
| ss6'CL | DIMTWGFDY | 9 | I |
| s5A9'CL | SNWYWYFDL | 9 | III |
| NEWM | NLIAGCIDV | 9 | II |
| L2A12'CL | GGKGGEFDD | 9 | I |
| B5G10H'CL | DSGNYRIDY | 9 | II |
| E55 3.9'CL | DPRLDAFDI | 9 | III |
| SpAl-29'CL | GYSYPVWGR | 9 | III |
| AM28'CL | LVGNSWLDY | 9 | III |
| BM2'CL | DL?GLVVEY | 9 | III |
| CM29'CL | KVSLSAFDI | 9 | III |
| B-B10 M0'CL | RGDAMYFDV | 9 | I |
| HSVCBM8'CL | DPNPWYFDL | 9 | III |
| HSVCD53'CL | DYGDYAFDI | 9 | III |
| HSVCBG6'CL | SAHSDAFDM | 9 | III |
| MICA 4'CL | LEGLGWFDP | 9 | I |
| 1/11'CL | RSDYGAIDY | 9 | III |
| 5/8'CL | NLGFYHMDV | 9 | III |
| B6204'CL | EARGGGGEY | 9 | III |
| VH CLONE 1'CL | EGWISALNG | 9 | III |
| VH CLONE 32'CL | EGEGEYFDY | 9 | III |
| MG6-1'CL | ERTSGDFDF | 9 | III |
| MG6-3'CL | NSPGATFES | 9 | III |
| Daudi'CL | GNGQKCFDY | 9 | III |
| IE4'CL | RGSLQYLDY | 9 | I |
| IF10'CL | NNGSYYFDY | 9 | I |
| hsighvm148'C L | GSDYSNFAY | 9 | III |
| E3-MPO'CL | STHRSAFDV | 9 | II |
| rev9Fd'CL | EGVHKNFDH | 9 | III |
| NANUC-1'CL | LSRAGGFDI | 9 | III |
| Patient | RMPAVAFDY | 9 | II |
| 14'CL | | | |
| 14G1'CL | RMPAVAFDY | 9 | II |
| 14G2'CL | RMRAVAFDY | 9 | II |
| 14G3'CL | RMPAVAFDY | 9 | II |
| A15'CL | DYGGNPAEL | 9 | I |
| G15'CL | GPTCSGGSC | 9 | I |
| M11'CL | RKGAAHFDY | 9 | I |
| RF-DI1'CL | EEVGGYFQH | 9 | III |
| AC-18'CL | DFDGGSFDY | 9 | III |
| AC-29'CL | DFDGGSLDY | 9 | III |
| AC-40'CL | DFDGGSFDY | 9 | III |
| TR35'CL | KVPSHGMDY | 9 | III |
| TR36'CL | KVPSHGMDY | 9 | III |
| TR37'CL | KVPSHGMDY | 9 | III |
| TR38'CL | KVPSHGMDY | 9 | III |
| L34'CL | QPLARHFDP | 9 | III |
| L100'CL | GPLMRWFDD | 9 | III |
| WG1'CL | VAVAGGFDP | 9 | III |
| RF-ET5'CL | GVEVAGTAS | 9 | I |
| RF-ET10'CL | YYESSAGPP | 9 | III |
| EW-D1'CL | EIPRGGSCY | 9 | III |
| EW-D3'CL | EIPRGGSCY | 9 | III |
| KN-D6'CL | KEKWDSSRC | 9 | III |
| HH-M2'CL | GSAAAGTQG | 9 | III |
| AK-D8'CL | DFSWAGPHF | 9 | III |
| BALL-1'CL | GTHYYDIRV | 9 | III |
| YJ | DGSGSYEGN | 9 | III |
| K2.2 | GGAVAAFDY | 9 | III |
| E2.5 | KPVTGGEDY | 9 | III |
| MSL5 | DYDGAWFAY | 9 | I |
| Hb-2 | WDGRLLVDY | 9 | III |
| b4'CL | HKGLRYFDY | 9 | III |
| b3'CL | HKGLRYFDY | 9 | III |
| b2'CL | HKGLRYFDY | 9 | III |
| b5'CL | HKGLRYFDY | 9 | III |
| b17'CL | HKGLRYFDY | 9 | III |
| b19'CL | HKGLRYFDY | 9 | III |
| A3-H2'CL | YRGDTYDYS | 9 | III |
| A3-M9'CL | WVGATTSDY | 9 | III |
| Tmu69'CL | EDMDYGMDV | 9 | III |
| Amu1d4-3'CL | GGRDRYLVY | 9 | III |
| 1946'CL | VRVSYGMDV | 9 | V |
| GN901v1.0 | MRKGYAMDY | 9 | III |
| GN901v1.1 | MRKGYAMDY | 9 | III |
| N901H/KOL | MRKGYAMDY | 9 | III |
| N901H/G36005 | MRKGYAMDY | 9 | III |
| N901H/PLO123 | MRKGYAMDY | 9 | III |
| Patient 14'CL | RMPAVAFDY | 9 | II |
| 14G1(2)'CL | RMPAVAFDY | 9 | II |
| 14G2'CL | RMRAVAFDY | 9 | II |
| 14G3'CL | RMPAVAFDY | 9 | II |
| CLL-8'CL | TSIVRGFGP | 9 | II |
| BA-1F'CL | DFFRDYFDY | 9 | I |
| BA-2P'CL | DFFRDYFDY | 9 | III |
| L3055 4.6'CL | GGTQPFDIR | 9 | II |
| 15'CL | SQASGPFDY | 9 | I |
| CL-G'CL | GLYQLLFDY | 9 | III |
| CL-O'CL | AGGRTSFDP | 9 | I |
| BA3'CL | EGNTKAPDY | 9 | III |
| PS'CL | NGTSGDFDY | 9 | II |
| HNK20 hu7 | YGTSYWFPY | 9 | I |
| HNK20 hu10 | YGTSYWFPY | 9 | I |
| Amu1d4-3'CL | GGRDRYLVY | 9 | III |
| Amu1e10-3'CL | LRYQLLYNY | 9 | I |
| 1e8-3'CL | YIAYDAFDI | 9 | I |
| 1f7-3'CL | ITPRNAVDI | 9 | III |
| Agamma41-3'CL | DGLLAATDY | 9 | III |
| Agamma8-3'CL | DRAYLDFWG | 9 | III |
| Amu10-3'CL | DKEPAYFDY | 9 | I |
| Amu2-1'CL | RGFNGQLIF | 9 | I |
| Amu40-2'CL | LSVVVPAAL | 9 | III |
| Amu70-1'CL | LADDDPEDF | 9 | I |
| Tmu69'CL | EDMDYGMDV | 9 | III |
| B7-g2B01'CL | SAGGSAWST | 9 | III |
| B8-g3C11'CL | DRSYYGMDV | 9 | III |
| B8-g3F05'CL | DKGTRYSDQ | 9 | III |
| BF1N-g3C12'CL | WLVEGSFDY | 9 | III |
| BF1N-g3H05'CL | GYVGSSLDY | 9 | III |
| BF1P-g2A11'CL | WHQLRGPDY | 9 | III |
| BF2P1-g3D10'CL | ENSDYYFDY | 9 | III |
| BF2P1-g3E12'CL | DGTYGSGVR | 9 | III |
| BF2P2-g3C10'CL | GGSMVPFDY | 9 | III |
| BF2P2-g3D05'CL | RGWNYYFDS | 9 | III |
| BF2P2-g3D12'CL | DAYYYGLDV | 9 | III |
| BF2P3-g3C10'CL | DGRYDPIDY | 9 | III |
| BF2P1-g7B02'CL | VGSSGWYDY | 9 | I |
| BF2N1-g1C10'CL | DLYDYYDEP | 9 | I |
| BF2N2-g1A11'CL | DGAAASFDY | 9 | I |
| BF2N2-g1E01'CL | WGADYFDY | 9 | I |
| BF2N1-g3F03'CL | DQNWGYFDY | 9 | III |
| BF2N2-g3B07'CL | GVLRDAFDI | 9 | III |
| BF2N2-g3C03'CL | ASDGYGMDV | 9 | III |
| BF2N2-g3F07'CL | GVLRHALDI | 9 | III |
| BF2N1-g4A03'CL | GGCGWYKNY | 9 | III |
| BF2N1-g4B10'CL | GSNYAKTGY | 9 | III |
| BF2N1-g4C11'CL | GKFQLLFDY | 9 | III |
| BF2N1-g6A07'CL | ALHGGGMDV | 9 | III |
| BF2N1-g6F07'CL | ALHGGGMDV | 9 | II |
| BF2N2-g6D09'CL | VYPPDAFDL | 9 | III |
| mAbRWL1'CL | PWDYWFFDL | 9 | II |
| SV-10 | DRVAAAGDY | 9 | III |
| SV-7 | DKGTRYSDQ | 9 | III |
| SV-9 | DRVATIPDY | 9 | III |
| DN6'CL | ERGITLMDV | 9 | I |
| DN7'CL | ERGITLMDV | 9 | I |
| SC12'CL | LDWLLPIDY | 9 | I |
| SC13'CL | LDWLLPIDY | 9 | I |
| D11'CL | DDGDRAFGY | 9 | III |
| JON'CL | DPWPAAFDI | 9 | III |
| DEZ'CL | VRGSWSGDS | 9 | III |
| BAR | RHSSDWYPY | 9 | III |
| KC13H'CL | SSPYGALDY | 9 | III |
| clone 15'CL | GLDQYKTGH | 9 | II |
| B22'CL | GAGAAPHDY | 9 | III |
| P13'CL | GAGAAPHDY | 9 | III |
| PS'CL | NGTSGDFDY | 9 | II |
| Patient 2 | ALRPATFDF | 9 | III |

**Table 2**

| # Name | L3 | Length-L3 | Class |
|---|---|---|---|
| HIV-s8'CL | QQYADLIT | 8 | IGG1-KAPPA |
| FOG1-A4'CL | QQYYSTPT | 8 | -KAPPA |
| SA-4B'CL | QQYNTYPT | 8 | IGG-KAPPA |
| HIV-b5'CL | QQGNSFPK | 8 | IGG1-KAPPA |
| HIV-loop8'CL | QQYGYSLT | 8 | IGG1-KAPPA |
| Reg-A'CL | QQFGGSFT | 8 | KAPPA |
| 9F12Fab'CL | QQSSNTVT | 8 | KAPPA |
| GP68'CL | QQYNSLIT | 8 | IGG1-KAPPA |
| C471'CL | QQYNNWPT | 8 | IGM-KAPPA |
| B8807'CL | LQHNSYPF | 8 | IGM-KAPPA |
| B122'CL | QQYNSQYT | 8 | IGM-KAPPA |
| B6204'CL | QQYGSLWT | 8 | IGM-KAPPA |
| IM-9'CL | QHYNRPWT | 8 | IGG-KAPPA |
| T48.16-G8'CL | QQYGSRLT | 8 | KAPPA |
| 7F'CL | QHYGTPRT | 8 | IGG1-KAPPA |
| 1A6'CL | QQYNNWPT | 8 | IGM-KAPPA |
| 1.69'CL | MQATQFPT | 8 | IGM-KAPPA |
| antiTac | HQASTYPL | 8 | KAPPA |
| WE | QQYGRSPR | 8 | KAPPA |
| D1.1 | QQDDLPYT | 8 | KAPPA |
| K2.2 | QNDNLPLT | 8 | KAPPA |
| E1.1 | QQESLPLT | 8 | KAPPA |
| E2.4 | QQDNLPLT | 8 | KAPPA |
| E2.5 | QQESLPCG | 8 | KAPPA |
| E2.11 | QQDSLPLT | 8 | KAPPA |
| SSaPB | QQYGSSRS | 8 | IGM-KAPPA |
| SEGaBM | QQYGSSRT | 8 | IGM-KAPPA |
| SELcLN | QQYCGSLS | 8 | IGM-KAPPA |
| mAb5.G3'CL | QQSYSTLT | 8 | IGM |
| P7'CL | QLYGSSLT | 8 | KAPPA |
| PA | QQYNNLWT | 8 | KAPPA |
| CAR | QQYNTFFT | 8 | KAPPA |
| Taykv322'CL | QQYGSSPT | 8 | KAPPA |
| Taykv310'CL | QQYGSSLT | 8 | KAPPA |
| Taykv320'CL | QQYGSSLT | 8 | KAPPA |
| slkv22'CL | QQYGSSKT | 8 | KAPPA |
| LES | QQYNNWPP | 8 | KAPPA |
| RF-TMC1'CL | QHRNNWPP | 8 | IGM-KAPPA-III |
| slkv4'CL | QQRSNWPS | 8 | KAPPA |
| MD3.13'CL | QQYGSSPT | 8 | KAPPA |
| VJI'CL | QQYDTIPT | 8 | KAPPA |
| rsv13L'CL | QASINTPL | 8 | IGG1-KAPPA |
| II.2'CL | MQALQPWT | 8 | KAPPA |
| I.75'CL | QQGFSDRS | 8 | KAPPA |
| II.14'CL | MQATQFVT | 8 | KAPPA |
| III.7'CL | QRCKGMFS | 8 | KAPPA |
| SPA3-16'CL | QQYGGSPW | 8 | KAPPA |
| VL CLONE 52'CL | CRSHWPYT | 8 | KAPPA |
| 6F5-01'CL | QQYYSTPP | 8 | KAPPA |
| 6F7-42'CL | QQCNTNPP | 8 | KAPPA |
| 6F8-01'CL | QQYYSTPP | 8 | KAPPA |
| 6F9-31'CL | QQYYSVPP | 8 | KAPPA |
| D7'CL | QQYDSLVT | 8 | IGG1-KAPPA |
| HuVK'CL | HQYLSSWT | 8 | KAPPA |
| BC-26'CL | MQGIHLLT | 8 | KAPPA |
| VkLaE34'CL | QHYYGTPH | 8 | KAPPA |
| FL9-K | QQYNTYPT | 8 | KAPPA |
| HSC7 | QEFGDSGT | 8 | IGG |
| HSC28 | QQYGGSPW | 8 | IGG |
| SEGcPB | QQYGSSRT | 8 | IGM-KAPPA |
| P3'CL | QQYDSLPT | 8 | KAPPA |
| A5K3'CL | QQYGSVFT | 8 | IGM |
| BZ1K1'CL | QQYNSYCS | 8 | IGM |
| BZ2K1'CL | QQYYSTPL | 8 | IGM |
| D11K3'CL | QQYNDWPT | 8 | IGM |
| D17K2'CL | MQNIQFPT | 8 | IGM |
| F21K1'CL | QQYDNLPP | 8 | IGM |
| F22K3'CL | QLLR?LRT | 8 | IGM |
| SCFV198'CL | YQYNNGYT | 8 | KAPPA |
| KC25L'CL | QQRSNWPT | 8 | KAPPA |
| ASSYN13'CL | QQYGTSHT | 8 | KAPPA |
| BCPBL1'CL | QQYNHWPS | 8 | KAPPA |
| BCPBL4'CL | QQYGSLYT | 8 | KAPPA |
| BCPBL6'CL | QQNKDWPL | 8 | KAPPA |
| BCSYN6'CL | QQFGTSLT | 8 | KAPPA |
| ITPBL14'CL | QQRSNWWT | 8 | KAPPA |
| ITPBL2'CL | QQCSNWPT | 8 | KAPPA |
| SP10'CL | QQYGSSPT | 8 | KAPPA |

**Table 3**

| # Name | L3 | Length-L3 | Class |
|---|---|---|---|
| 8E10'CL | QQYGSSPSIT | 10 | IGM-KAPPA |
| III-2R'CL | QKYNSAPPST | 10 | IGM-KAPPA |
| II-1'CL | QEYNNWPLWT | 10 | KAPPA |
| 35G6'CL | QQYGGSPPWT | 10 | IGM-KAPPA |
| GF4/1.1'CL | HEYNGWPPWT | 10 | IGG3-KAPPA |
| RF-TS5'CL | QQYNSYSPLT | 10 | IGM-KAPPA |
| O-81'CL | MQHTHWSPIT | 10 | IGM-KAPPA |
| mAb114'CL | QHYNNWPPWT | 10 | IGM-KAPPA |
| HIV-B8'CL | QQSYNTPPWT | 10 | IGG1-KAPPA |
| HIV-b8'CL | QQSYNTPPWT | 10 | IGG1-KAPPA |
| TT117'CL | QHYGSSPPWT | 10 | IGG1-KAPPA |
| HIV- loop13'CL | QQHNNWPPLT | 10 | IGG1-KAPPA |
| HIV-s3'CL | QVYGQSPVFT | 10 | IGG1-KAPPA |
| 1-185-37'CL | QQYGSSPMYT | 10 | IGM-KAPPA |
| 1-187-29'CL | QQYGSSPMYT | 10 | IGM-KAPPA |
| HIV-s5'CL | QRFGTSPLYT | 10 | IGG1-KAPPA |
| HIV-b3'CL | QQYGDSPLYS | 10 | IGG1-KAPPA |
| GER | QQYDDWPPIT | 10 | IGG-KAPPA |
| BLI'CL | QQLNSYPPYT | 10 | IGM-KAPPA |
| 2A4'CL | QQSYSTPPDT | 10 | IGG |
| 0-16'CL | QHYNNWPPSS | 10 | KAPPA |
| mAb48'CL | QHYNRLPPWT | 10 | IGG3-KAPPA |
| 447.8H'CL | QQYDRSVPLT | 10 | KAPPA |
| GP13'CL | QQYYTTPTYT | 10 | IGG1-KAPPA |
| M37GO37'CL | QQYYTTPPLT | 10 | IGG-KAPPA |
| 9500'CL | QQLYSYPHLT | 10 | IGM-KAPPA |
| 9702'CL | CQQYGSSRWT | 10 | IGG-KAPPA |
| GSD2B5B10'CL | MQALQTPMST | 10 | KAPPA |
| MD2F4'CL | QQRSEWPPLT | 10 | KAPPA |
| GAN4B.5'CL | QQYDTSPAWT | 10 | KAPPA |
| NANUC-2'CL | QQYGSSQGFT | 10 | IgG1-kappa |
| SOL10'CL | MQSIQLPRWT | 10 | KAPPA |
| AB1/2'CL | QHYGLSPPIT | 10 | IGG1-KAPPA |
| AB4'CL | QEYGSSPPRT | 10 | IGG1-KAPPA |
| RH-14'CL | SSYRSSSTRV | 10 | IGG1-LAMBDA |
| AB1/2'CL | QHYGLSPPIT | 10 | IGG1-KAPPA |
| AB4'CL | QEYGSSPPRT | 10 | IGG1-KAPPA |
| L55-81'CL | QQYYTTLPLT | 10 | IGM-KAPPA |
| B3 | SSYSSTTRVV | 10 | IGG |
| HUL-mRF'CL | QQYGSSPQTF | 10 | IGM-KAPPA |
| 25C1'CL | FCQYNRYPYT | 10 | KAPPA |
| LC4aPB | LQRSNWGEVT | 10 | IGM-KAPPA |
| LC4bPB | QQRSNWGEVT | 10 | IGM-KAPPA |
| LC4cPB | QQRSNWGEVT | 10 | IGM-KAPPA |
| mAb3.B6'CL | QQYGSSPLFT | 10 | IGM |
| mAb1.C8'CL | CSYTSSSTLV | 10 | IGM |
| P9'CL | QQRSNWPPIT | 10 | KAPPA |
| 21H9'CL | QQSYNTLSLT | 10 | IGG1-KAPPA |
| 19A5'CL | QHYGNSPPYT | 10 | IGG1-KAPPA |
| 43F10'CL | QQSHKTLAWT | 10 | IGG1-KAPPA |
| FON'CL | MQGTYWPPYT | 10 | IGM-KAPPA |
| Hu PR1A3 | HQYYTYPLFT | 10 | KAPPA |
| hu PR1A3 | HQYYTYPLFT | 10 | KAPPA |
| CLL-412'CL | QQSYSTPPWT | 10 | IGG-KAPPA |
| MEV | QQSYTNPEVT | 10 | KAPPA |
| SON | QQYGSSPPYT | 10 | IGM-KAPPA |
| HEW''CL | QQYGSSPRYT | 10 | KAPPA |
| HEW'CL | QQYGSSPRYT | 10 | KAPPA |
| JH' | QQFGNSPPL? | 10 | IGG2-KAPPA |
| HG2B10K'CL | QQYAGSPPVT | 10 | IGG-KAPPA |
| CLL'CL | QQYNNWPPWT | 10 | IGM-KAPPA |
| slkv12'CL | QQYNNWPPWT | 10 | KAPPA |
| bkv6'CL | QQRSNCSGLT | 10 | KAPPA |
| slkv11'CL | QQYNNWPPWT | 10 | KAPPA |
| slkv13'CL | QQYNNWPPWT | 10 | KAPPA |
| bkv7'CL | QQYNNWPPCT | 10 | KAPPA |
| bkv22'CL | QQYNNWPPWT | 10 | KAPPA |
| bkv35'CL | QQRSFWPPLT | 10 | KAPPA |
| MD3.3'CL | QQRSNWPSIT | 10 | KAPPA |
| MD3.1'CL | QQRSNWPPLT | 10 | KAPPA |
| GA3.6'CL | QQRTNWPIFT | 10 | KAPPA |
| M3.5N'CL | QQRSNWPPGT | 10 | KAPPA |
| MD3.4'CL | QQYNNWPPLT | 10 | KAPPA |
| M3.1N'CL | QQYNNWPTWT | 10 | KAPPA |
| GA3.4'CL | QQRMRWPPLT | 10 | KAPPA |
| MD3.7'CL | QQYGSSPKWT | 10 | KAPPA |
| MD3.9'CL | QQYGSSPQYT | 10 | KAPPA |
| GA3.1'CL | QQYGSSPPYT | 10 | KAPPA |
| bkv32'CL | QQYDRSLPRT | 10 | KAPPA |
| GA3.5'CL | QQYGNSPLFS | 10 | KAPPA |
| GA3.8'CL | QQYGGSPLFS | 10 | KAPPA |
| E29.1 KAPPA'CL | QQYNNWPTWT | 10 | IGM-KAPPA |
| R5A3K'CL | MQALQTLGLT | 10 | IGM-KAPPA |
| R1C8K'CL | MQALQTLGLT | 10 | IGG-KAPPA |
| I.24'CL | QQSHSAPPYT | 10 | KAPPA |
| III.12'CL | QQYGSSPLFT | 10 | KAPPA |
| III.5'CL | QQYNDWPPWT | 10 | KAPPA |
| I.18'CL | QQYNGNSPLT | 10 | KAPPA |
| I.67'CL | QQLNTYPPWT | 10 | KAPPA |
| III.6'CL | HKYGGSPPYT | 10 | KAPPA |
| II.65'CL | MQDTHWPPWT | 10 | KAPPA |
| III.14'CL | QHYGRSPPLT | 10 | KAPPA |
| 424.F6.24'CL | QQYGNSPPYT | 10 | KAPPA |
| T33-5'CL | QQYGSSPPYT | 10 | IGM-KAPPA |
| AL-MH | QQYFNVPPVT | 10 | KAPPA |
| AL-Es305 | QHYHNLPPTT | 10 | KAPPA |
| L47'CL | IQGTHWPQYT | 10 | IGM-KAPPA AND LAMBDA |
| F29'CL | QQYGSSRALT | 10 | IGM-KAPPA AND LAMBDA |
| G28'CL | QQYYSTPSYT | 10 | IGM-KAPPA AND |
| | | | LAMBDA |
| G21'CL | MQALQTLMCS | 10 | IGM-KAPPA AND LAMBDA |
| VL CLONE 45'CL | QQSYSTPPLT | 10 | KAPPA |
| VL CLONE 48'CL | QQSYSTPPIT | 10 | KAPPA |
| VL CLONE 56'CL | QQYGGSLPIT | 10 | KAPPA |
| C9'CL | QQYGSSTPLT | 10 | IGG1-KAPPA |
| ITC88'CL | QQRSSWPPLT | 10 | KAPPA |
| AC18'CL | QQRYSWPPLT | 10 | KAPPA |
| AC31'CL | QQRYNWPPLT | 10 | KAPPA |
| AC32'CL | QQRSNWPPLT | 10 | KAPPA |
| AC37'CL | QQRSSWPPLT | 10 | KAPPA |
| B'20 | QQYNNWPPWT | 10 | IgM-VkIIIa |
| (Humkv328-Jk1)'CL | | | |
| B9601 (Vg-Jk2)'CL | QQRSNWPPYT | 10 | IgM-VkIIIa |
| MF8 | QQYNNWPPWT | 10 | IgM-VkIIIa |
| (Humkv328-Jk1)'CL | | | |
| B'2 | QQYNNWPPWT | 10 | IgM-VkIIIa |
| (Humkv328-Jk1)'CL | | | |
| kappa1'CL | QQYGSSPPIT | 10 | IGG2-KAPPA |
| kappa2'CL | QQYNNWPPIT | 10 | IGG2-KAPPA |
| kappa3'CL | QQRSSWPPIT | 10 | IGG2-KAPPA |
| kappa4'CL | QQYGSSPRVT | 10 | IGG2-KAPPA |
| kappa5'CL | QQYNTNSPIS | 10 | IGG2-KAPPA |
| kappa7'CL | QNYGSSPRIT | 10 | IGG2-KAPPA |
| kappa8'CL | QQYGSSPPIT | 10 | IGG2-KAPPA |
| ToP218'CL | MQSIQLPRFT | 10 | KAPPA |
| ToP241'CL | MQSVQLPRFT | 10 | KAPPA |
| ToP309'CL | MQSVQLPRFT | 10 | KAPPA |
| L1236K3'CL | QQYDKWPPVT | 10 | KAPPA |
| SOL1'CL | MQSIQFPRWT | 10 | KAPPA |
| BC-2'CL | MQGIHLPPYI | 10 | KAPPA |
| P3'CL | NQGTQWLLYT | 10 | KAPPA |
| P5'CL | QQYNSYAPYT | 10 | KAPPA |
| AB1/2'CL | QHYGLSPPIT | 10 | IGG-KAPPA |
| AB4'CL | QEYGSSPPRT | 10 | IGG-KAPPA |
| MH | QQYFNVPPVT | 10 | KAPPA |
| FL6-K | QQLTSYPPWT | 10 | KAPPA |
| FL2-K | QQVNSYPGLT | 10 | KAPPA |
| FL4-K | QQVFSYPGIT | 10 | KAPPA |
| FL1-K | QQYTSLPGIT | 10 | KAPPA |
| MM4-K | QHSYSTLPLT | 10 | KAPPA |
| MM9-K | QQYYNIPYIT | 10 | KAPPA |
| HSC4 | QLYGSSPRVT | 10 | IGG |
| HSC11 | QQYANWPPIT | 10 | IGG |
| HSC13 | QQYNISPRDT | 10 | IGG |
| HSC23 | QQFGSSPLIT | 10 | IGG |
| HSC35 | QQYGDFPRVT | 10 | IGG |
| REV | QQYGDWPPYT | 10 | KAPPA |
| BLU | QQYYTTLSWT | 10 | KAPPA |
| BK2'CL | QQYNKWPPLT | 10 | KAPPA |
| GK6'CL | MQGTHWLPVT | 10 | IGG-KAPPA |
| L1236K3'CL | QQYDKWPPVT | 10 | KAPPA |
| P1'CL | QQYDNLPPIH | 10 | KAPPA |
| H01'CL | QQLNNYPPFT | 10 | KAPPA |
| I01'CL | QQSYSTPPYT | 10 | KAPPA |
| I10'CL | QQSYSTPPYS | 10 | KAPPA |
| I12'CL | QQSYSTPPYT | 10 | KAPPA |
| 126TP14K'CL | QQYNNWLPFT | 10 | IGG-KAPPA |
| L32'CL | AAWDDSLTLM | 10 | IGM-KAPPA |

For insertion of CDR3s, single oligonucleotides encoding each of the CDR3s of table H from the plus strand were synthesised with 12 homologous nucleotides added to each termini for annealing to the consensus VH and VL genes. In addition to these CDR sequences, CDRs from the antibody E25 (see example 4) were included. These primers were extended and secondary primers were added to introduce directly adjacent to the N and C termini of the VH and VL genes (without C regions) 5'NotI-3'XbaI sites for VH and 5'SpeI-3'BamHI for VL. Prior to cloning, a further pair of complimentary primers was used to insert the linker sequence (Gly4Ser)3 between VH and VL whilst maintaining XbaI and SpeI sites. Full-sized VH-linker-VL fragments were digested with NotI and BamHI and were cloned into NotI-BamHI digested pBluescript II KS(+/-) (Stratagene, Amsterdam, Netherlands).

Individual Bluescript clones were picked, plasmid DNA was purified and dispensed robotically into 96 well plates as described in WO99/11777. DNAs were then subjected to IVTT including tRNA-biotinyl-lysine and further robotically arrayed onto a streptavidin surface as described in WO99/11777. The immobilised initial scFv library of 10,000 independant clones was then screened by incubation with recombinant human IgE Fab (see example 4). Wells were blocked with PBS/3% BSA at room temperature for 1 hour, washed three times in PBS and treated with 5ug/ml human IgE Fab in PBS/3% BSA for 1 hour. Wells were then washed a further three times in PBS and treated with 5ug/ml alkaline phosphatase-labelled chimeric anti-IgE (example 4) in PBS/3% BSA for 1.5hrs. Wells were further washed five times in PBS and colour developed using the substrates 5-bromo-1-chloro-3-indolyl phosphate and nitro blue tetrazolium (Roche Molecular) for visualization. A strong signal observed at a frequency of 1 of 9600 wells was shown to derive from a VH and VL pair both containing E25 CDR3's.

### Example 6-Construction of Composite Mouse Anti-TNFa Antibody

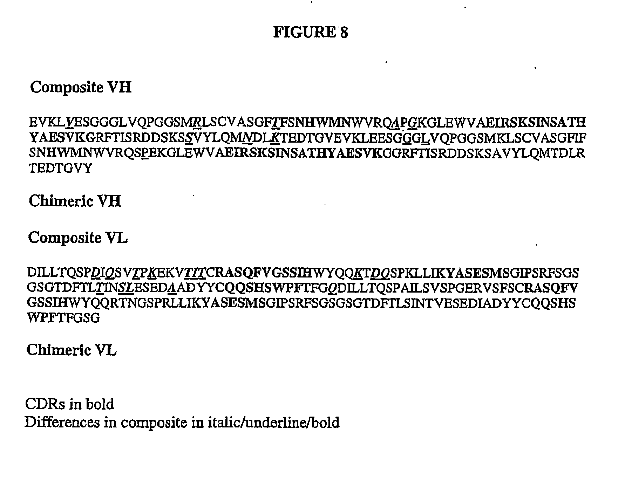

A mouse variable region sequence library was created as described in example 1 for the human library using NCBI Igblast, Kabat and Genbank databases. The reference antibody variable region sequences used was a chimeric anti-TNFα antibody known as Remicade® (Le et al., US6277969) using the variable regions of the mouse cA2 antibody. Segments from the in silico mouse variable region sequence library were selected partly corresponding amino acids in the Remicade® variable region but including variations designed to avoid human T cell epitopes in the sequence in the form of non-self human MHC class II binders measured as in example 1. Composite mouse VH and VL sequences compared to sequences used in the chimeric antibody are shown in figure 8 indicating differences of 9 and 16 amino acids in VH and VL respectively between the two antibodies as a result of segment selection for epitope avoidance in the Composite Mouse antibody.

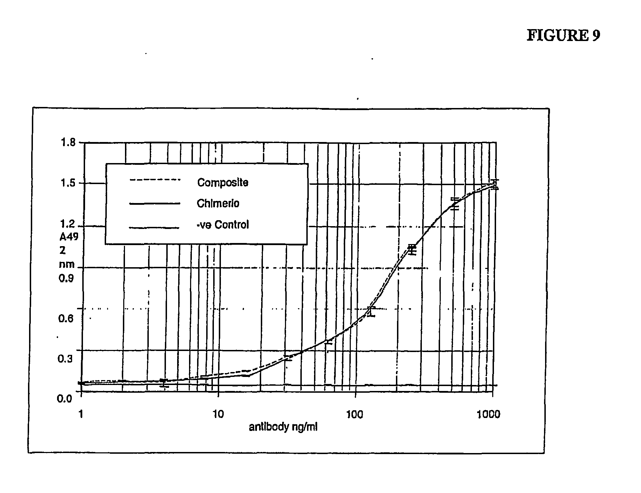

The Composite Mouse and chimeric anti-TNFα antibodies were generated as described in example 1. Comparison of purified antibodies for binding to immobilised human TNFα in a standard ELISA (described in WO 03/042247A2) showed that the Composite Mouse antibody retained the full binding capacity of the chimeric anti-TNFα antibody (figure 9). The immunogenicity of these antibodies was then compared as described in example 2 using 24 HLA-DR typed human blood samples for T cell assays. The results showed that the chimaeric anti-TNFα antibody induced significant proliferative responses (SI greater than 2) in nine of the twenty four healthy donors tested (37.5%) compared to the Composite Mouse anti-TNFα antibody where none of the twenty four donors (0%) induced SI>2. These results indicated that a Composite Mouse antibody comprising segments of variable region sequence derived totally from mouse V regions with selection of such segments to avoid human T cell epitopes could remove the immunogenicity in human T cell assays displayed by the corresponding chimeric antibody without any epitope avoidance measures.

### Example 7: Construction of a Composite Human Anti-TNFa Antibody

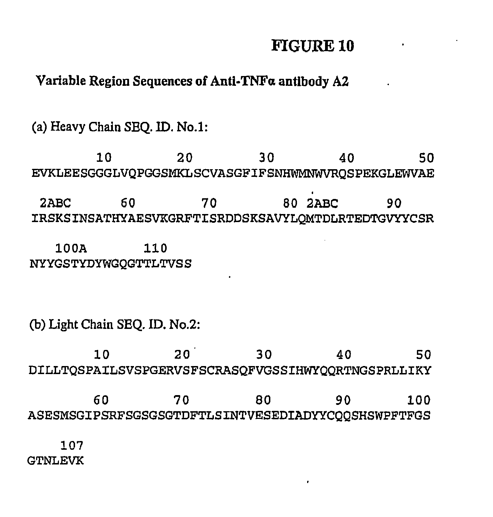

The reference mouse variable region heavy and light chain sequences of antibody A2 directed against human TNFα was obtained from US patent 5656272 (Fig. 10. SEQ. IDs No. 1 and No. 2 respectively). A structural model was made of the mouse reference variable regions and amino-acids critical for CDR conformation were identified based upon their distance from the CDRs (<3Ǻ) and their likely packing close to CDRs. Important, but less critical residues were identified based upon their distance from the CDRs (>3Ǻ <6Ǻ) and their likely influence on more critical residues packing closer to the CDRs. A further set of residues were identified based upon their frequency of occurrence in mouse antibody sequences i.e. amino-acids found at a particular location with a frequency of less than 1%.

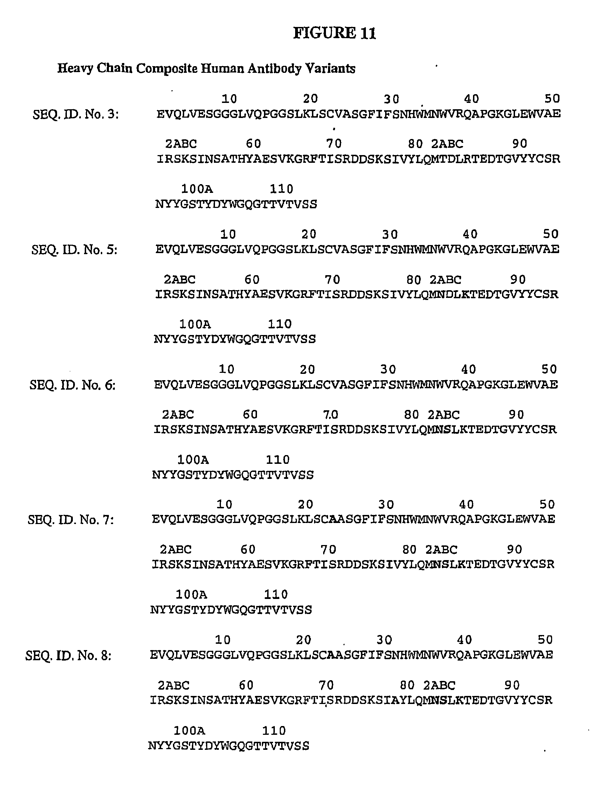

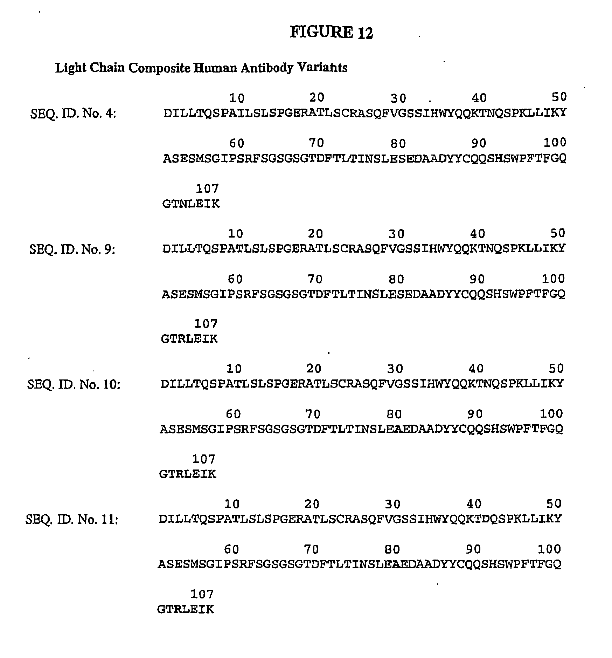

Human V region sequence segments that included as many of these residues as possible were selected (table 4) to create full-length VH and VL sequences. Alterations were made to these sequences to include all the identified structurally important residues to create sequences to serve as a template for epitope avoidance and Composite Human Antibody design. A preferred sequence for each composite VH and VL was designed to include important residues from the reference mouse antibody. These variable heavy and light chain amino acid sequences are shown in Figs. 11 and 12. SEQ IDs No. 3 and No. 4 respectively.

**Table 4: Human Antibody Database Derivation of Sequence Segments For Primary CHAB Variants**

| **(a) Heavy Chain** | |
|---|---|
| Genbank Accession No. | Sequence segment |
| CAA61442 | EVQLVESGGGLVQPGGSLKLSC |
| CAD88676 | LSCVASGFIFS |
| CAB37182 | FSNHWM |
| AAS86088 | HWMNWVRQAPGKGLEWVA |
| CAC43592 | AEI |
| ABB54411 | IRSKS |
| AAL96548 | SIN |
| AAK51359 | NSA |
| CAA67405 | SAT |
| CAB87447 | ATHYA |
| AAD30769 | HYAESVKGRFTISRD |
| CAC 15703 | RFTISRDDSKSI |
| AAQ05509 | IVYLQM |
| AAT96742 | YLQMTDLR |
| AAD20526 | LRTEDTGVYYC |
| CAB44788 | VYYCSRNY |
| AA038724 | NYYGS |
| AAK14004 | GSTY |
| AAD20470 | TYDYWGQGT |
| AAB32435 | DYWGQGTTVTVSS |

| **(b) Light Chain** | |
|---|---|
| Genbank Accession No. | Sequence segment |
| CAC06686 | DILLTQ |
| AAX57564 | LTQSPAILSLSPGERATLSC |
| X72820 | LSLSPGERATLSCRASQ |
| AAC15439 | QFV |
| AAZ09058 | VGSS |
| Z84907 | SSI |
| AAL10835 | IHWYQQK |
| AAQ21835 | QQKPNQSPKLLIK |
| M27751 | LLIKYAS |
| AAY16612 | YASE |
| AAR89591 | ES |
| AAD19534 | SM |
| AAV71416 | MSG |
| AAZ09098 | GIP |
| CAG27043 | PSRFSGSGSGTDFTLTINSLE |
| AAQ21937 | SLESEDAA |
| AAC41988 | ADYYCQQ |
| AAY33370 | YYCQQSHS |
| AAD19457 | HSWP |
| AAQ55271 | WPFTFGQGT |
| AAW69118 | TFGQGTNLEIK |

The composite heavy and light chain variable region sequences were scanned for the presence of potential T cell epitopes using a variety of *in silico* methods (e.g. Propred [http://imtech.res.in/raghava/propred/index.html], Peptide Threading [www.csd.abdn.ac.uk/ gjlk/MHC-thread], SYFPEITHI (www.syfpeithi.de), MHCpred (www.jenner.ac.uk/MHCPred/) and compared to homologous human germ-line framework region sequences in conjuction with reference mouse CDRs.

The following heavy chain variable region variants were made (see Fig 11):
SEQ. ID. No. 5 contains the following changes with respect to SEQ. ID. No. 3: T82aN + R83K.
SEQ. ID. No. 6 contains the following changes with respect to SEQ. ID. No. 3: T82aN + R83K + D82bS
SEQ. ID. No. 7 contains the following changes with respect to SEQ. ID. No. 3: T82aN + R83K + D82bS + V23A.
SEQ. ID. No. 8 contains the following changes with respect to SEQ. ID. No. 3: T82aN + R83K + D82bS + V23A + V78A

The following light chain variable region variants were made (see Fig. 12):
SEQ. ID. No. 9 contains the following changes with respect to SEQ. ID. No. 4: I10T + N103R.
SEQ. ID. No. 10 contains the following changes with respect to SEQ. ID. No. 4: I10T + N103R + S80A.
SEQ. ID. No. 11 contains the following changes with respect to SEQ. ID. No. 4: I10T + N103R + S80A + N41D.

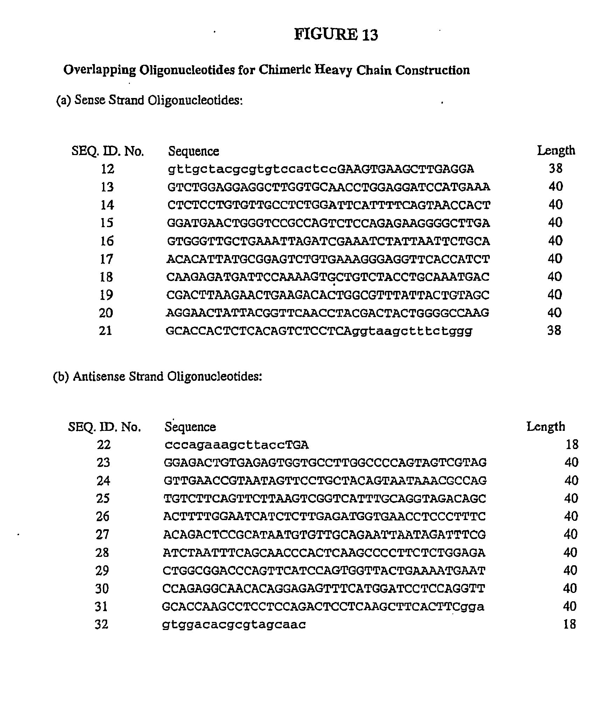

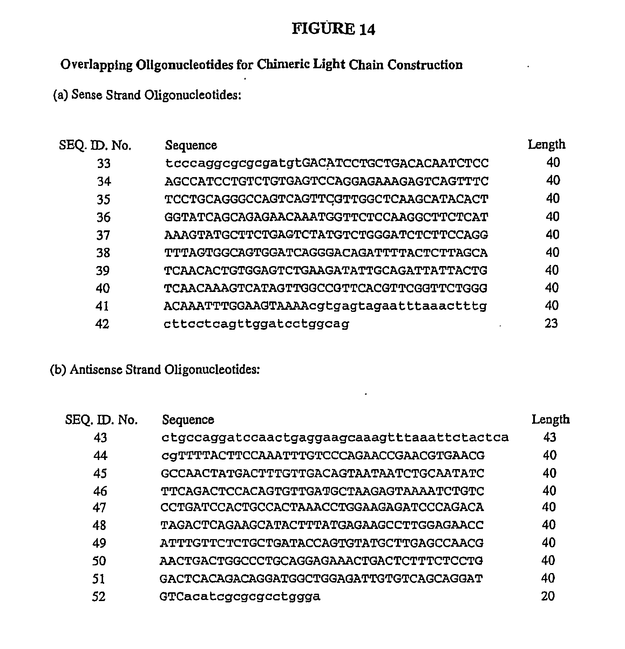

For construction of a control chimeric antibody, the nucleotide sequences that translate to give SEQ. IDs No. 1 and No. 2 were constructed using a series of overlapping 40mer synthetic oligonucleotides. The V region sequences were flanked by additional 5' and 3' sequences to facilitate cloning into mammalian expression vectors. The sequences of the oligonucleotides are shown in Figure 13 and Figure 14 Oligonucleotides were purchased from Sigma-Genosys (Poole, UK) and resuspended at a concentration of 100µM. 1µl of each of the heavy chain sense strand oligonucleotides, except the most 5' oligonucleotide, were mixed together and 1.5µl (approx. 1µg) of the mix was treated with Polynucleotide Kinase (PNK, Invitrogen, Paisley UK) in a 20µl reaction containing additionally: 2µl 10x PNK buffer, 2µl 10mM ATP, 14µl H₂O 0.5µl (5 units) PNK. The reaction was incubated at 37°C for 30 min and the enzyme inactivated by heating at 70°C for 20 min. The heavy chain antisense, light chain sense and antisense oligonucleotides were similarly phosphorylated. The 5' oligonucleotide from each set was diluted 1 in 9 with H₂O and 1.5µl added to the appropriate reaction mix. Each reaction was then diluted to 0.5ml and spin dialyzed in an Amicon microcon YM3 concentrator for 90 min at 8000 rpm until the volume was not more than 44µl.

The sense and antisense mixes for the heavy chain, and those for the light chain, were combined and made up to 88µl with H₂O. 10µl 10x Ligase Chain Reaction (LCR) buffer and 2µl Pfu ligase (8 units, Stratagene, Cambridge UK) were added to each reaction and incubated as follows in a programmable heating block: 94°C for 4 min, then 60°C for 3min for 1 cycle followed by 20 cycles of 94°C for 39 sec. then 60°C for 2 min. Finally the reactions were incubated for 5 min at 60°C. 10µl of each LCR was run through a 1% agarose gel stained with ethidium bromide and compared to 1Kb ladder markers (Invitrogen). A smear of ligated DNA was observed in each lane, surrounding a faint specific band of approximately 400bp.

The heavy and light chain LCRs were amplified via PCR using as primers SEQ. ID. No.s 12 and 22 for the heavy chain and SEQ. ID. No.s 33 and 43 for the light chain. The following were included in each reaction: 5µl LCR, 5µl 10x Expand HiFi buffer (Roche, Lewes UK), 1µl 10mM NTP mix, 0.25µl each primer (from 100µM stocks), 0.5µl Expand HiFi polymerase (3 units, Roche) and 38µl H₂O. The reactions were cycled as follows: 94°C 2 min followed by 20 cycles of 94°C for 30 sec, 60°C for 30 sec and 72°C for 30 sec. Finally the reaction was incubated for 5 min at 72°C. The yield and specificity of the reaction was confirmed by agarose gel electrophoresis, as above. Specific, sharp bands at approximately 400bp were observed for each reaction.

The reaction products were purified using a Qiagen PCR purification kit and each eluted in 30µl H₂O. The heavy chain product was digested in a standard reaction with restriction enzymes *Mlu* I and *Hind* III and the light chain product was digested with *Bss*H II and *Bam*H I. The reaction products were again purified using a Qiagen PCR purification kit and each eluted in 30µl H₂O.

The light chain expression vector pANT08 was based upon a pAT153 backbone and contains in the following order: CMV immediate/early enhancer promoter -590 to +7, a 30nt 5' UTR derived from a highly expressed mouse antibody light chain RNA, a mouse consensus light chain signal sequence incorporating a *Bss*H II restriction site near the variable region start codon, a short linker (in place of a variable region) to a human composite intron contining 33 nt from the variable region splice site to a *Bam*H I restriction site followed by a fragment of human genomic DNA containing 343 nt of the intron preceding the human constant Kappa (CK) region gene, the CK gene and CK polyA.

The heavy chain expression vector pANT09 was similar to pANT08 through the promoter region, which is followed by: a 62nt 5' UTR derived from the heavy chain counterpart of that described above, a mouse heavy chain consensus signal sequence that incorporates a *Mlu* I restriction site near the variable region start codon, a short linker (in place of a variable region) to the variable region splice site immediately followed by a fragment of human genomic DNA from a *Hind* III restriction site located in the intron 211 nt upstream of the CH1 gene, to the end of the CH region poly A site. This fragment includes the CH1, hinge, CH2 and CH3 introns and exons of human IgG1. This vector also included a gene for dihydrofolate reductase, controlled by an SV40 promoter and polyA signal, for resistance to methotrexate.

2µg each vector was digested with the relevant restriction enzymes in standard reactions in a total volume of 30µl. Each reaction was run through a 1% agarose gel, as above, and the vector specific bands (6.0Kbp heavy chain and 4.2Kbp light chain) were excised from the gel and purified using a Qiagen gel extract kit and eluted in 30µl H₂O.

1µl each digested vector was ligated to 3µl of the corresponding digested variable gene PCR product using a Ligafast kit (Promega, Southampton UK). 2.5µl each ligation reaction was transformed into sub-cloning efficiency competent XL1-blue (Stratagene), as instructed by the manufacturer, and plated onto LB agar plates containing 100µg/ml ampicillin and incubated overnight at 37°C. Ten bacterial colonies from each ligation were inoculated into 10ml 2x YT broth containing 100µg/ml ampicillin and grown overnight at 37°C with shaking. Plasmid was purified from 1.5ml each overnight culture using a Qiagen plasmid preparation kit and each eluted in 50µl H₂O. The plasmids were sent to a contract sequencing facility and sequenced with a standard CMV promoter primer and clones with the correct V region gene sequence identified.

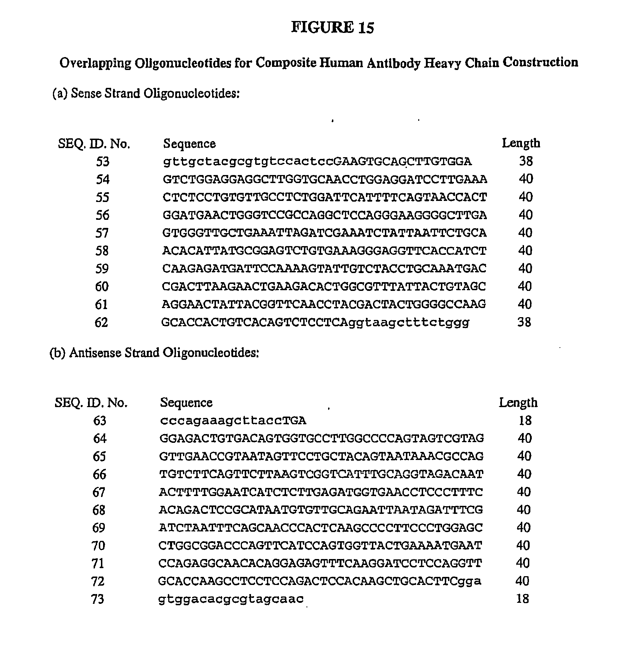

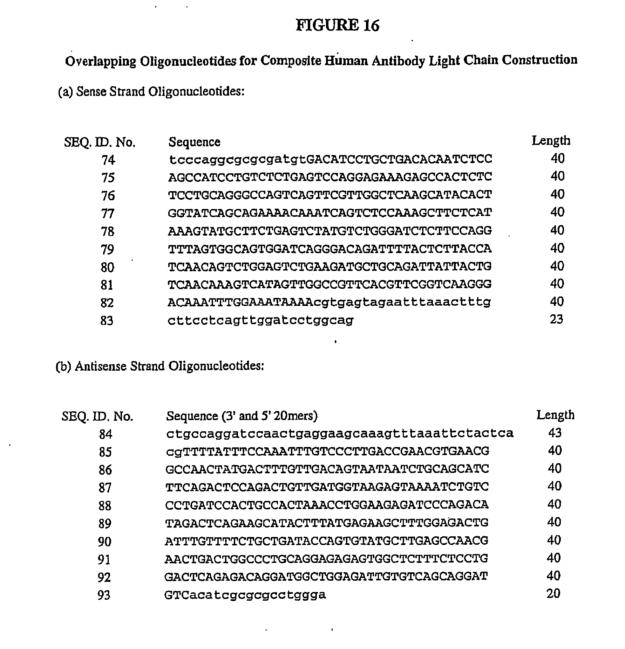

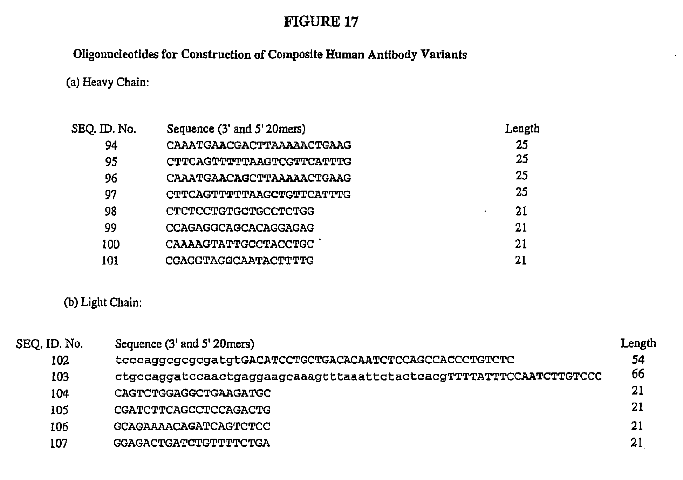

For construction of Compsoite Human Antibodies, the nucleotide sequences that translate to give SEQ. IDs No. 3 and No. 4 were constructed using a series of overlapping 40mer synthetic oligonucleotides. The sequences of the oligonucleotides are shown in Figure 15 and Figure 16. The nucleotide sequence that translates to give SEQ. ID. No. 5 was constructed via overlap PCR using oligonucleotide primers SEQ. ID. No.s 94 and 95 (Figure 17) together with oligonucleotides SEQ. ID. No.s 53 and 63 and the plasmid DNA of the primary Composite Human Antibody heavy chain variant as template. Two PCR reactions were done using as primer pairs either SEQ. ID. No.s 53 and 95, or SEQ. ID. No.s 94 and 63. The following were included in each reaction: 1µl (100ng) plasmid template, 5µl 10x Expand HiFi buffer (Roche), 1µl 10mM NTP mix, 0.25µl each primer (from 100µM stocks), 0.5µl Expand HiFi polymerase (3 units, Roche) and 42µl H₂O. The reactions were cycled as follows: 94°C 2 min followed by 20 cycles of 94°C for 30 sec, 60°C for 30 sec and 72°C for 30 sec. Finally the reaction was incubated for 5 min at 72°C. The entire reactions were electrophoresed through a 1% agarose gel and the specific bands of 295bp and 126bp were excised and purified using a Qiagen gel extraction kit. The DNAs were eluted in 30µl H₂O.

The two purified fragments were joined in a PCR reaction using oligonucleotide primers SEQ. ID. No.s 53 and 63 using PCR conditions as described above, except that the template used was 1µl 295bp product and 1µl 126bp product, hence the amount of H₂O was reduced to 41µl. The joined PCR product of 396bp was purified using a Qiagen PCR purification kit and was eluted in 30µl H₂O.

The nucleotide sequence that translates to give SEQ. ID. No. 6 was constructed via overlap PCR using oligonucleotide primers SEQ. ID. No.s 96 and 97 (Figure 17) together with oligonucleotides SEQ. ID. No.s 53 and 63 and the plasmid DNA of the primary Composite Human Antibody heavy chain variant as template. Two PCR reactions were done using as primer pairs either SEQ. ID. No.s 53 and 97, or SEQ. ID. No.s 96 and 63. The first stage PCRs were done as described above and yielded fragments of 295bp and 126bp. These fragments were purified, joined together and repurified, also as described above.

The nucleotide sequence that translates to give SEQ. ID. No. 7 was constructed via overlap PCR using oligonucleotide primers SEQ. ID. No.s 98 and 99 (Figure 17) together with oligonucleotides SEQ. ID. No.s 53 and 63 and the PCR product for SEQ. ID. No. 6 as template. Two PCR reactions were done using as primer pairs either SEQ. ID. No.s 53 and 99, or SEQ. ID. No.s 98 and 63. The first stage PCRs were done as described above and yielded fragments of 98bp and 318bp. These fragments were purified, joined together and repurified, also as described above.

The nucleotide sequence that translates to give SEQ. ID. No. 8 was constructed via overlap PCR using oligonucleotide primers SEQ. ID. No.s 100 and 101 (Figure 17) together with oligonucleotides SEQ. ID. No.s 53 and 63 and the PCR product for SEQ. ID. No. 7 as template. Two PCR reactions were done using as primer pairs either SEQ. ID. No.s 53 and 101, or SEQ. ID. No.s 100 and 63. The first stage PCRs were done as described above and yielded fragments of 270bp and 155bp. These fragments were purified, joined together and repurified, also as described above.

Each of the above PCR products was digested with *Mlu* I and *Hind* III and ligated into similarly digested pANT09. The ligations were transformed and plated, colonies picked, plasmids prepared and sequenced all as described above.

The nucleotide sequence that translates to give SEQ. ID. No. 9 was constructed via PCR using oligonucleotide primers SEQ. ID. No.s 102 and 103 (Figure 17) and the plasmid DNA of the primary Composite Human Antibody light chain variant as template.A single PCR reaction was done, as described for the heavy chain variants, that yielded a product of 383bp. The entire reaction was electrophoresed through a 1% agarose gel and the specific band was excised and purified using a Qiagen gel extraction kit. The DNA was eluted in 30µl H₂O.

The nucleotide sequence that translates to give SEQ. ID. No. 10 was constructed via overlap PCR using oligonucleotide primers SEQ. ID. No.s 104 and 105 (Figure 17) together with oligonucleotides SEQ. ID. No.s 74 and 84 and the PCR product for SEQ. ID. No. 9 as template. Two PCR reactions were done using as primer pairs either SEQ. ID. No.s 74 and 105, or SEQ. ID. No.s 104 and 84. The first stage PCRs were done as described above for the heavy chain variants and yielded fragments of 265bp and 139bp. These fragments were purified, joined together to create a product of 383bp and repurified, also as described above for the heavy chain variants.

The nucleotide sequence that translates to give SEQ. ID. No. 11 was constructed via overlap PCR using oligonucleotide primers SEQ. ID. No.s 106 and 107 (Figure 17) together with oligonucleotides SEQ. ID. No.s 74 and 84 and the PCR product for SEQ. ID. No. 10 as template. Two PCR reactions were done using as primer pairs either SEQ. ID. No.s 74 and 107, or SEQ. ID. No.s 106 and 84. The first stage PCRs were done as described above for the heavy chain variants and yielded fragments of 148bp and 256bp. These fragments were purified, joined together to create a product of 383bp and repurified, also as described above for the heavy chain variants.

Each of the above PCR products was digested with *Bss*H II and *Bam*H I and ligated into similarly digested pANT08. The ligations were transformed and plated, colonies picked, plasmids prepared and sequenced all as described above.

CHO-K1 cells (ATCC# CCL-61) were propagated in high glucose DMEM containing 10% FCS, L-glutamine, sodium pyruvate and L-proline. Near confluent cultures were harvested for transfection using Lipofectamine 2000 as instructed by the manufacturer (Invitrogen). Transfections were done in 48 well plates seeded with 200µl cells at 3x10⁵ cells/ml using a total of 0.5µg plasmid DNA comprising 0.3µg heavy chain construct and 0.2µg light chain construct.

Transfections were incubated at 37°C/5% CO₂ for 48 to 72h before harvesting the supernatants. Antibody expression was quantified by ELISA using: a mouse monoclonal anti-human IgG capture antibody, human IgG1/Kappa standards and HRP conjugated goat anti-human Kappa light chains as detection antibody (all reagents from Sigma).

All combinations of heavy and light chains were transfected (i.e. 6 heavy chains x 5 light chains = 30 transfections). Antibody expression levels were generally in the range of 0.5 to 2.0 µg/ml, however no expression was observed with heavy chain SEQ. ID. No. 8.

The expressed antibodies were tested for their ability to neutralize the activity of human TNFα using TNF-sensitive WEHI-164 cells (Espevik et al., J. Immunol. Methods 1986, 95, 99-105). Cells were plated in 1 µg/ml actinomycin D at 5x10⁴ cells per well in 96-well microtiter plates for 3-4 hours. Cells were exposed to 40 pM human TNF and varying concentrations of the chimeric antibody (range 1ng/ml to 500ng/ml) to create a standard curve. The various combinations of heavy and light chains were tested at a single concentration point of 25ng/ml that had previously been determined as the ED₅₀ of the chimeric antibody. All assays were done in triplicate. The mixtures were incubated overnight at 37°C. Cell viability was determined by adding 3-[4,5-dimethyl-thiazol-2-yl]-2, 5-diphenyltetrazoliumbromide dye (MTT) to a final concentration of 0.5 mg/ml, incubating for 4 hours at 37°C, lysing the cells in 0.1M HCl, 0.1% SDS and measuring the optical density at 550 nm wavelength.

The optical densities from the heavy and light chain combinations were used to calculate the apparent antibody concentrations from the standard curve. The apparent concentration of the chimeric was divided by that of each of the variant combinations to give a fold difference value. Values lower than that for the chimeric indicated that those combinations were more effective at protecting the cells from TNFα cytotoxicity, whereas higher values indicated that they were less effective. The values for all combinations are shown in Table 5.

**Table 5: Ratio of Activities of Composite Human Antibody Variants compared to Chimeric Antibody**

| **SEQ. ID. No.** | **1** | **3** | **5** | **6** | **7** | **8** |
|---|---|---|---|---|---|---|
| **2** | 1.00 | 1.38 | 1.24 | 1.20 | 1.02 | ND |
| **4** | 1.51 | 2.28 | 1.28 | 1.38 | 1.05 | ND |
| **9** | 1.28 | 2.14 | 1.32 | 1.77 | 0.95 | ND |
| **10** | 1.31 | 2.51 | 1.17 | 1.63 | 0.98 | ND |
| **11** | 16.90 | 15.15 | 196.49 | 134.08 | 105.61 | ND |

The following Composite Human Antibody heavy and light chain combinations gave fold differences close to 1.0: SEQ. ID. No.s 5/10, SEQ. ID. No.s 7/4, SEQ. ID. No.s 7/9, SEQ. ID. No.s 7/10. These combinations were selected for further study.

The expression plasmids carrying the sequences selected above were transfected into NS0 cells (ECACC No. 85110503). The cells were grown in high glucose DMEM containing L-glutamine, sodium pyruvate, 5% ultra low IgG FCS and pen/strep. Cells were harvested during log phase of growth, spun down and resuspended at 5x10⁶ cells/ml in fresh growth media. 750µl cells were mixed with a total of 30µg of each plasmid pair, which had been linearised with *Ssp* I, in 50µl H₂O. The cell/plasmid mixture was transferred to a 4mm gap cuvette and electroporated using an Equibio Easyject Plus at 250V, 1500µF, infinite resistance. The electroporate was immediately transferred to 25ml pre-warmed growth media and plated out in 5x 96 well flat bottomed plates at 100µl/well. The plates were incubated at 37°C/5% CO₂. 48h post-electroporation, 50µl media containing 300nM methotrexate was added to each well to give a final concentration of 100nM. 7 days post-electroporation a further 50µl of media containing 100nM methotrexate was added to each well.

Approximately 2 week post-electroporation, the media in some wells began to turn yellow, indicating transfected colony growth. Media from these wells were tested for antibody expression using the anti-human IgG Fc capture/ anti-human Ig Kappa light chain HRP conjugate detection ELISA. The test samples were compared to a human IgG1/Kappa standard and antibody expression levels estimated. Colonies expressing useful amounts of antibody were expanded in media containing 200nM methotrexate.

Antibodies were purified from 500ml culture media via protein A affinity chromarography followed by size exclusion chromatography using Sephacryl S200. The purified antibodies were quantified by UV absorbance at 280nm, assuming that OD₂₈₀ 1 = 1.4mg/ml.

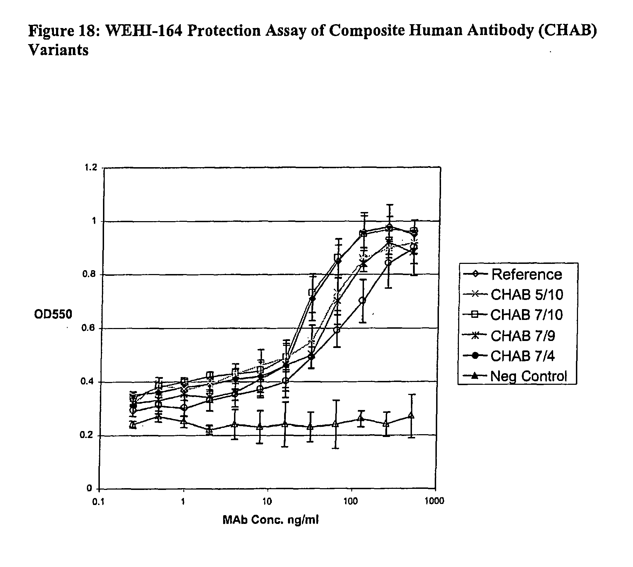

Purified chimeric and composite antibodies were tested for activity via the WEHI-164 protection assay described in example 4 above. Each antibody was tested over the full concentration range previously used to create the standard curve (see Figure 18). Composite Human Antibody 7/10 (i.e. containing SEQ. ID. No.s 7 and 10) was found to be the most active variant and had the same activity as the chimeric antibody. Composite Human Antibodies 7/9 and 5/10 had similar activity that was slightly reduced compared to the chimeric, and Composite Human Antibody 7/4 was the least active.

Therefore since Composite Human Antibody 7/10 was predicted to have the most favourable MHC class II binding profile and was the most active variant, this was selected for testing in a time course T cell proliferation assay. Human PBMCs were prepared from buffy coats derived from human blood donations via two rounds of Ficoll density centrifugation. The prepared PBMC were resuspended at a density of 3x10⁷ cells/ml in 1ml aliquots in 90% human AB serum/10% DMSO, and stored under liquid nitrogen. PBMC were tissue typed using a Dynal Allset® PCR typing kit.

The lead Composite Human Antibody was compared to the chimeric antibody in whole protein T cell assays using human PBMC from 20 healthy donors. PBMC from each donor were thawed, washed and resuspended in AIM V serum free lymphocyte growth media. On day 1, 50µg protein was added to 2ml bulk cultures of 4x10⁶ PBMC in 24 well plates, and triplicate 100µl aliquots were removed and transferred to 96 well plates on days 6 to 9. Each aliquot was pulsed with 75µl media containing 1µCi tritiated thymidine for 24h, before harvesting and measuring incorporation of radioactivity. Results were normalised by calculation of the Stimulation Index (SI).

Coverage of a wide range of HLA DR allotypes was achieved by selecting donors according to individual MHC haplotypes.

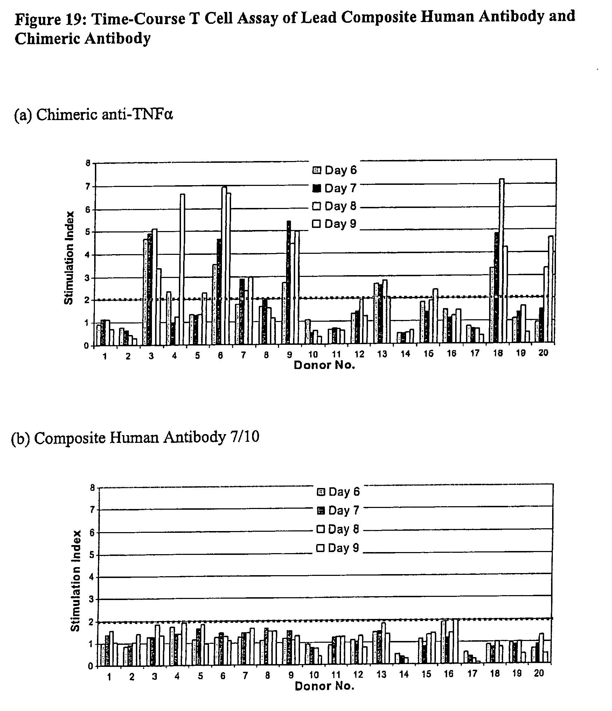

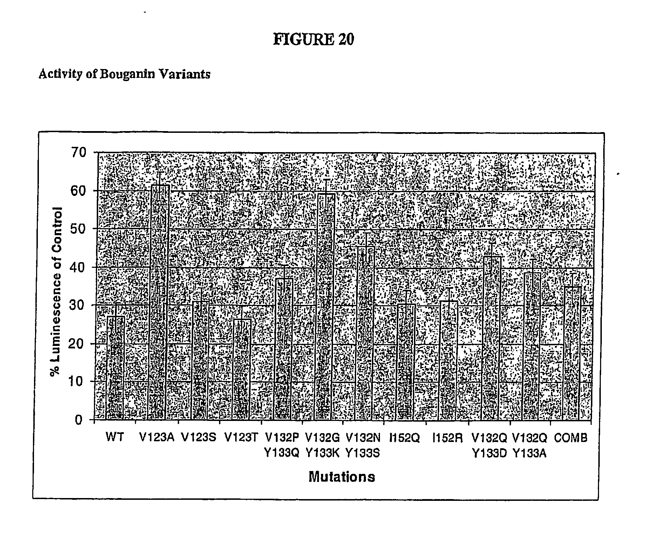

The results of the time-course assay are shown in Figure 19 and demonstrated that the chimeric antibody (Figure 19(a)) elicits a T cell response (SI >= 2) on at least one day in 10 of the 20 donors. In contrast, Composite Human Antibody (Figure 19(b)) failed to elicit a response in any of the donors at any time point. Therefore a non-immunogenic Composite Human Antibody was successfully constructed from segments of human antibodies using a mouse anti-TNFα antibody (A2) as reference.

## Claims

1. A method for screening a composite antibody variable region or antigen-binding fragment to avoid T cell epitopes, wherein the method comprises the following steps:
Generating a library of genesencoding composite antibody variable regions or antigen-binding fragments derived from multiple peptides of amino acid sequence of 2 to 31 amino acids long from other antibodies or antigen-binding fragments, wherein the two or more peptides are neither whole CDRs nor whole framework regions;
Screening the antibody variable region or antigen-binding fragment to avoid T cell epitopes; and
Expressing and screening the library of antibody variable regions or antigen-binding fragments for binding to one or more antigens of interest.

2. A method as claimed in claim 1 wherein the two or more peptides used to generate the library are derived from human antibodies.

## Patentansprüche

1. Verfahren zum Screening einer variablen Region oder eines Antigen bindenden Fragments eines zusammengesetzten Antikörpers zur Vermeidung von T-Zell-Epitopen, wobei das Verfahren die folgenden Schritte umfasst:
das Erzeugen einer Bibliothek von Genen, die für variable Regionen oder Antigen bindende Fragmente von zusammengesetzten Antikörpern kodieren, abgeleitet von einer Vielzahl an Peptiden mit einer Aminosäuresequenz mit einer Länge von 2 bis 31 Aminosäuren, aus weiteren Antikörpern oder Antigen bindenden Fragmenten, wobei es sich bei den zwei oder mehreren Peptiden weder um vollständige komplementaritätsbestimmende Regionen (CDRs) noch um vollständige Gerüstregionen handelt;
das Screening der variablen Regionen oder der Antigen bindenden Fragmente der Antikörper zur Vermeidung von T-Zell-Epitopen; und
das Exprimieren und Screening der Bibliothek aus variablen Regionen oder Antigen bindenden Fragmenten der Antikörper im Hinblick auf die Bindung an eines oder mehrere relevante Antigene.

2. Verfahren nach Anspruch 1, wobei die zwei oder mehreren Peptide, die zur Erzeugung der Bibliothek verwendet werden, von humanen Antikörpern abgeleitet sind.

## Revendications

1. Procédé de criblage d'une région variable d'anticorps composite ou d'un fragment de liaison à l'antigène pour éviter des épitopes de cellules T, dans lequel le procédé comprend les étapes suivantes :
la génération d'une bibliothèque de gènes codant des régions variables d'anticorps composite ou des fragments de liaison à l'antigène dérivés de multiples peptides de séquence d'acides aminés longues de 2 à 31 acides aminés provenant d'autres anticorps ou fragments de liaison à l'antigène, dans lequel les deux peptides ou plus ne sont pas des CDR (région déterminante de la complémentarité) entières, ni des régions d'ossature entières ;
le criblage de la région variable d'anticorps ou du fragment de liaison à l'antigène pour éviter des épitopes de cellules T ; et
l'expression et le criblage de la bibliothèque de régions variables d'anticorps ou de fragments de liaison à l'antigène pour liaison à un ou plusieurs antigènes d'intérêt.

2. Procédé selon la revendication 1, dans lequel les deux peptides ou plus utilisés pour générer la bibliothèque sont dérivés d'anticorps humains.
